(19) Europäisches Patentamt / European Patent Office / Office européen des brevets

(11) EP 3 763 393 A1

(12) # EUROPEAN PATENT APPLICATION
published in accordance with Art. 153(4) EPC

(43) Date of publication:
**13.01.2021 Bulletin 2021/02**

(21) Application number: **19756605.2**

(22) Date of filing: **25.02.2019**

(51) Int Cl.:
***A61K 48/00** (2006.01)* ***A61K 9/127** (2006.01)*
***A61P 43/00** (2006.01)* ***C12N 15/11** (2006.01)*
***C12N 15/12** (2006.01)* ***C12N 15/88** (2006.01)*
***C12Q 1/6883** (2018.01)* ***G01N 33/53** (2006.01)*

(86) International application number:
**PCT/JP2019/007145**

(87) International publication number:
**WO 2019/164003 (29.08.2019 Gazette 2019/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.02.2018 JP 2018031349**

(71) Applicant: **LUCA Science Inc.**
**Chuo-ku**
**Tokyo 103-0023 (JP)**

(72) Inventors:
- **HARASHIMA, Hideyoshi**
**Sapporo-shi, Hokkaido 060-0808 (JP)**
- **YAMADA, Yuma**
**Sapporo-shi, Hokkaido 060-0808 (JP)**
- **SOMIYA, Kana**
**Sapporo-shi, Hokkaido 060-0808 (JP)**
- **OSAKA, Hitoshi**
**Shimotsuke-shi, Tochigi 329-0498 (JP)**

(74) Representative: **J A Kemp LLP**
**14 South Square**
**Gray's Inn**
**London WC1R 5JJ (GB)**

(54) **NUCLEIC ACID FOR EXPRESSING PROTEIN IN MITOCHONDRIA, LIPID MEMBRANE STRUCTURE ENCAPSULATING SAID NUCLEIC ACID, AND USE THEREOF**

(57) The present invention relates to a mitochondria-targeted lipid membrane structure encapsulating a nucleic acid represented by any of the following a) to d):
a) an RNA comprising, in this order, a nucleotide sequence of a first mitochondrial tRNA, a nucleotide sequence of an mRNA encoding a target protein, and a nucleotide sequence of a second mitochondrial tRNA, wherein the nucleotide sequence of the mRNA has one or more UGAs as a tryptophan codon;
b) a DNA comprising a nucleotide sequence of a promoter and a nucleotide sequence complementary to the RNA of a);
c) an RNA comprising a nucleotide sequence of an mRNA encoding a target protein, and a nucleotide sequence of a poly(A) chain present at the 3' end side thereof, wherein the nucleotide sequence of the mRNA has one or more UGAs as a tryptophan codon, AUG as a start codon, and UAA as a stop codon; and
d) a DNA comprising a nucleotide sequence of a promoter and a nucleotide sequence complementary to the RNA of c).

FIG. 1

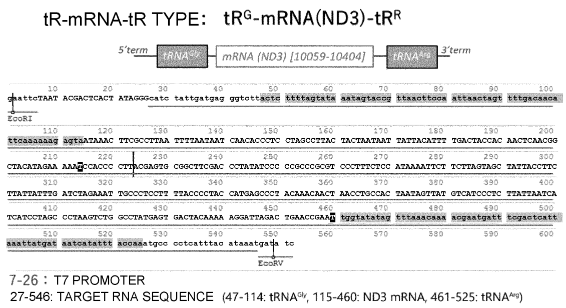


7-26: T7 PROMOTER
27-546: TARGET RNA SEQUENCE (47-114: tRNA^Gly, 115-460: ND3 mRNA, 461-525: tRNA^Arg)



Printed by Jouve, 75001 PARIS (FR)

**Description**

Technical Field

The present invention relates to a nucleic acid for expressing a protein in mitochondria, a mitochondria-targeted lipid membrane structure encapsulating the nucleic acid, a use of the nucleic acid or the lipid membrane structure in a pharmaceutical composition or in the production of a cell preparation, and a primer DNA set for quantifying a point mutation of the mitochondrial protein ND3.

Background Art

Mitochondria, which are one of the organelles of the cell, contain mitochondrial proteins that are transcribed and translated from nuclear genomic DNA in the cell nucleus, then transported into mitochondria (mitochondrial proteins derived from nuclear DNA), and mitochondrial proteins that are encoded in mitochondrial genomic DNA independent of the cell nucleus, then transcribed and translated in mitochondria (mitochondrial proteins derived from mitochondrial DNA). Many associations between mutations in the genomic DNAs encoding these proteins and various diseases (encephalomyopathy, neurodegenerative diseases, cancer, diabetes, and the like) have been reported. These are collectively referred to as mitochondrial diseases. Hereinafter, the present invention will be described using human mitochondria as a representative example unless otherwise specified. Moreover, the term "mitochondrial protein" is used as a term including mitochondrial proteins derived from nuclear DNA and mitochondrial proteins derived from mitochondrial DNA, unless otherwise specified.

One of the methods for treating mitochondrial diseases that is currently under research and development is gene therapy, which aims to deliver into mitochondria proteins expected to have a therapeutic effect, for example, wild-type proteins corresponding to mutated mitochondrial proteins. In order to realize gene therapy, a foreign gene expression system that can transport a target protein transcribed in the cell nucleus and translated in the cytoplasm into mitochondria, or a foreign gene expression system that can directly express a target protein in mitochondria have been proposed.

A wide variety of expression vectors have been developed in connection with the transport of cytoplasmically expressed target proteins into mitochondria. Most of them utilize mitochondrial targeting signal peptides (MTS) contained in mitochondrial proteins derived from nuclear DNA. Specifically, an expression vector encoding a target protein having an MTS upstream (MTS-added protein) is delivered to the cell nucleus. The MTS-added protein is then expressed in the cytoplasm, and delivered to mitochondria.

This method may be useful for certain target proteins, but has the problem of poor applicability to mitochondrial proteins derived from mitochondrial DNA. This is because many mitochondrial proteins derived from mitochondrial DNA are insoluble in the cytoplasm, which causes the mitochondrial proteins derived from mitochondrial DNA expressed in the cytoplasm to aggregate and thus insufficiently migrate to mitochondria.

Patent Literature 1 discloses a method for suppressing the aggregation of a target protein in the cytoplasm by using an expression vector encoding an MTS-added protein consisting of an MTS having enhanced water solubility and a specific mitochondrial protein derived from mitochondrial DNA. However, mitochondrial proteins derived from mitochondrial DNA often show cytotoxicity when present in intracellular organelles other than mitochondria. Therefore, the range of target proteins for which the method described in Patent Literature 1 can be utilized is limited.

In addition, there remain concerns regarding the use of the above method utilizing an MTS-added protein as a tool for gene therapy, since it may cause fatal damage to the cells by interfering or competing with the intracellular transport of the original mitochondrial protein.

On the other hand, transcriptional expression of a necessary and sufficient amount of the target protein in mitochondria is first and foremost required in the use of a foreign gene expression system capable of directly expressing a target protein in mitochondria. In order to suit such a purpose, a promoter derived from a gene present in mitochondrial genomic DNA, for example, HSP (heavy strand promoter) is selected, and several expression vectors in which the DNA encoding the target protein is controlled by the promoter are designed. The expression vector is also devised to use a triplet codon frequently used in mitochondrial genomic DNA. However, the expression levels of these expression vectors have not reached the necessary and sufficient region for disease treatment so far.

For example, in Non Patent Literature 1, a method has been proposed in which DNA that is transcriptionally induced in mitochondria under HSP control is encapsulated in an artificial viral vector added with MTS, and the viral vector is directly introduced into mitochondria to express a target protein. This method has the advantage of having a relatively high expression level of the target protein from the introduced viral vector. However, aside from the safety problem resulting from the viral vector, whether the target protein is expressed in mitochondria as expected has not been completely verified.

As one of the foreign gene expression systems capable of directly expressing a target protein in mitochondria, the present inventors have proposed a promoter sequence exhibiting transcriptional activity in the cell nucleus of animal cells, and a recombinant expression vector having a coding region encoding a target protein containing one or more TGAs as a codon corresponding to tryptophan under the control of the promoter sequence (Patent Literature 2). Delivering this expression vector into mitochondria by utilizing a mitochondria-targeted lipid membrane structure allows to avoid undesirable translation of the target protein in the cytoplasm, and to further increase the expression level of the target protein in mitochondria. However, the need for efficient expression of target proteins in mitochondria remains high.

In the above conventional techniques, the nucleic acid delivered into mitochondria was exclusively DNA, but with the recent advances in RNA synthesis technology, a gene therapy strategy in which RNA instead of DNA is directly delivered into mitochondria has been proposed. A method applicable in a clinical setting that can deliver RNA encoding a target protein into mitochondria has, however, not yet been developed.

Citation List

Non Patent Literature

Non Patent Literature 1: Yu,H. et al., Proc. Natl. Acad. Sci. USA, 2012, 109: E1238-47

Patent Literature

Patent Literature 1: US 2015/0225740
Patent Literature 2: WO 2017/090763

Disclosure of the Invention

Technical Problem

The present invention aims to provide RNA and DNA for efficiently expressing a protein in mitochondria, and a method for introducing the RNA and DNA into mitochondria.

Solution to Problem

The present inventors have found that a target protein can be efficiently expressed in mitochondria by delivering a nucleic acid, particularly RNA, having certain characteristics on the nucleotide sequence, into mitochondria and completed the following invention.

(1) A mitochondria-targeted lipid membrane structure encapsulating a nucleic acid represented by any of the following a) to d) :

a) an RNA comprising, in this order, a nucleotide sequence of a first mitochondrial tRNA, a nucleotide sequence of an mRNA encoding a target protein, and a nucleotide sequence of a second mitochondrial tRNA, wherein the nucleotide sequence of the mRNA has one or more UGAs as a tryptophan codon;
b) a DNA comprising a nucleotide sequence of a promoter and a nucleotide sequence complementary to the RNA of a);
c) an RNA comprising a nucleotide sequence of an mRNA encoding a target protein, and a nucleotide sequence of a poly(A) chain present at the 3' end side thereof, wherein the nucleotide sequence of the mRNA has one or more UGAs as a tryptophan codon, AUG as a start codon, and UAA as a stop codon; and
d) a DNA comprising a nucleotide sequence of a promoter and a nucleotide sequence complementary to the RNA of c).

(2) The lipid membrane structure according to (1), wherein the nucleotide sequence of the mRNA and the nucleotide sequences of the two mitochondrial tRNAs of a) are contiguous.
(3) The lipid membrane structure according to (1), wherein the nucleotide sequence of the mRNA and the nucleotide sequence of the poly(A) chain of c) are contiguous.
(4) The lipid membrane structure according to any one of (1) to (3), wherein the target protein is a wild-type mitochondrial protein derived from mitochondrial DNA.
(5) The lipid membrane structure according to any one of (1) to (4), comprising dioleylphosphatidylethanolamine and sphingomyelin as constituent lipids of the lipid membrane.

(6) The lipid membrane structure according to any one of (1) to (5), which has a peptide consisting of the amino acid sequence set forth in SEQ ID NO: 1 on the lipid membrane surface.

(7) A pharmaceutical composition for treating and/or preventing mitochondrial diseases, the composition comprising as an active ingredient a nucleic acid represented by any of the following a) to d):

a) an RNA comprising, in this order, a nucleotide sequence of a first mitochondrial tRNA, a nucleotide sequence of an mRNA encoding a target protein, and a nucleotide sequence of a second mitochondrial tRNA, wherein the nucleotide sequence of the mRNA has one or more UGAs as a tryptophan codon;
b) a DNA comprising a nucleotide sequence of a promoter and a nucleotide sequence complementary to the RNA of a);
c) an RNA comprising a nucleotide sequence of an mRNA encoding a target protein, and a nucleotide sequence of a poly(A) chain present at the 3' end side thereof, wherein the nucleotide sequence of the mRNA has one or more UGAs as a tryptophan codon, AUG as a start codon, and UAA as a stop codon; and
d) a DNA comprising a nucleotide sequence of a promoter and a nucleotide sequence complementary to the RNA of c).

(8) The pharmaceutical composition according to (7), wherein the nucleotide sequence of the mRNA and the nucleotide sequences of the two mitochondrial tRNAs of a) are contiguous.

(9) The pharmaceutical composition according to (7),
wherein the nucleotide sequence of the mRNA and the nucleotide sequence of the poly(A) chain of c) are contiguous.

(10) The pharmaceutical composition according to any one of (7) to (9), wherein the target protein is a wild-type mitochondrial protein derived from mitochondrial DNA.

(11) The pharmaceutical composition according to any one of (7) to (10), wherein the nucleic acid is encapsulated in a mitochondria-targeted lipid membrane structure.

(12) A method for producing a cell preparation for treating and/or preventing mitochondrial diseases, the method comprising introducing *in vitro* the nucleic acid represented by any of the following a) to d) into cells derived from a patient with a mitochondrial disease or a person at risk of developing a mitochondrial disease:

a) an RNA comprising, in this order, a nucleotide sequence of a first mitochondrial tRNA, a nucleotide sequence of an mRNA encoding a target protein, and a nucleotide sequence of a second mitochondrial tRNA, wherein the nucleotide sequence of the mRNA has one or more UGAs as a tryptophan codon;
b) a DNA comprising a nucleotide sequence of a promoter and a nucleotide sequence complementary to the RNA of a);
c) an RNA comprising a nucleotide sequence of an mRNA encoding a target protein, and a nucleotide sequence of a poly(A) chain present at the 3' end side thereof, wherein the nucleotide sequence of the mRNA has one or more UGAs as a tryptophan codon, AUG as a start codon, and UAA as a stop codon; and
d) a DNA comprising a nucleotide sequence of a promoter and a nucleotide sequence complementary to the RNA of c).

(13) The production method according to (12), wherein the nucleotide sequence of the mRNA and the nucleotide sequences of the two mitochondrial tRNAs of a) are contiguous.

(14) The production method according to (12), wherein the nucleotide sequence of the mRNA and the nucleotide sequence of the poly(A) chain of c) are contiguous.

(15) The production method according to any one of (12) to (14), wherein the target protein is a wild-type mitochondrial protein derived from mitochondrial DNA.

(16) The production method according to any one of (12) to (15), wherein the nucleic acid is encapsulated in a mitochondria-targeted lipid membrane structure.

(17) An RNA comprising a nucleotide sequence of an mRNA encoding a target protein, and a nucleotide sequence of a poly(A) chain present at the 3' end side thereof, wherein the nucleotide sequence of the mRNA has one or more UGAs as a tryptophan codon, AUG as a start codon, and UAA as a stop codon.

(18) The RNA according to (17), wherein the nucleotide sequence of the mRNA and the nucleotide sequence of the poly(A) chain are contiguous.

(19) The RNA according to (17) or (18), wherein the protein is a wild-type mitochondrial protein derived from mitochondrial DNA.

(20) A DNA comprising a nucleotide sequence of a promoter and a nucleotide sequence complementary to the RNA defined in any one of (17) to (19).

(21) A kit for detecting T10158C, which is a point mutation of the mitochondrial ND3 gene, comprising:

a wild-type detection primer DNA set consisting of a combination of a primer DNA comprising the nucleotide sequence set forth in SEQ ID NO: 2 and a primer DNA consisting of the nucleotide sequence set forth in SEQ ID NO: 4, and

a mutant detection primer DNA set consisting of a combination of a primer DNA comprising the nucleotide sequence set forth in SEQ ID NO: 2 and a primer DNA consisting of the nucleotide sequence set forth in SEQ ID NO: 10.

(22) The kit according to (21), wherein the primer DNA comprising the nucleotide sequence set forth in SEQ ID NO: 2 is a primer DNA consisting of the nucleotide sequence set forth in SEQ ID NO: 3.

(23) The kit according to (21), wherein the primer DNA comprising the nucleotide sequence set forth in SEQ ID NO: 2 is a primer DNA consisting of the nucleotide sequence set forth in SEQ ID NO: 3 in which one or two nucleotides at the 5' end and/or the 3' end are deleted.

(24) The kit according to (21) or (23), wherein the primer DNA comprising the nucleotide sequence set forth in SEQ ID NO: 2 is a primer DNA consisting of the nucleotide sequence set forth in SEQ ID NO: 2.

Advantageous Effects of Invention

The lipid membrane structure and the nucleic acid of the present invention can express a target protein more efficiently and selectively in mitochondria, and therefore can be utilized as a safer and more effective medicament for treating mitochondrial diseases, or in the production of a cell preparation. In addition, the primer DNA set of the present invention can easily detect and quantify the mutation T10158C, which is a point mutation of the mitochondrial ND3 protein, by performing PCR using the primer DNA set.

Brief Description of Drawings

[Figure 1] Figure 1 is a diagram representing the structure of $tR^G$-mRNA(ND3)-$tR^R$, which is one aspect of the present invention, and the corresponding DNA nucleotide sequence.

[Figure 2] Figure 2 is a diagram representing the structure of ATG-mRNA(ND3)-polyA, which is one aspect of the present invention, and the corresponding DNA nucleotide sequence.

[Figure 3] Figure 3 is a calibration curve obtained by plotting the theoretical values of the mutation rate on the horizontal axis and the mutation rates calculated from the measured values by quantitative PCR on the vertical axis for the eleven primer DNA sets designed to detect the point mutation of the mitochondrial ND3 gene (T10158C) .

[Figure 4] Figure 4 is a calibration curve obtained by plotting the theoretical values of the mutation rate on the horizontal axis and the mean values ± standard errors of the mutation rates calculated from the measured values by three quantitative PCRs on the vertical axis for the primer DNA sets 7 and 11 shown in Figure 3.

[Figure 5] Figure 5 is a graph showing the changes in the T10158C mutation rate in cDNA complementary to mitochondrial ND3 mRNA when ATG-mRNA(ND3)-polyA/MITO is introduced into mitochondria of 7SP cells.

[Figure 6] Figure 6 is a graph showing the changes over time in the T10158C mutation rate in cDNA complementary to mitochondrial ND3 mRNA when ATG-mRNA(ND3)-polyA/MITO is introduced into mitochondria of 7SP cells.

[Figure 7] Figure 7 is a graph showing the changes over time in the T10158C mutation rate in cDNA complementary to mitochondrial ND3 mRNA when ATG-mRNA(ND3)-polyA/MITO is introduced into mitochondria of 7SP cells, and to mitochondrial ND3 mRNA when ATG-mRNA(ND3)-polyA is introduced with a commercially available RNA transfection reagent.

[Figure 8] Figure 8 is a graph showing the changes in the T10158C mutation rate in cDNA complementary to mitochondrial ND3 mRNA when ATG-mRNA(ND3)-polyA/MITO or $tR^G$-mRNA(ND3)-$tR^R$/MITO is introduced into mitochondria of 7SP cells, and to mitochondrial ND3 mRNA when ATG-mRNA(ND3)-polyA and $tR^G$-mRNA(ND3)-$tR^R$ are introduced with a commercially available RNA transfection reagent.

[Figure 9] Figure 9 is a diagram showing a time schedule for measuring mitochondrial respiration capacity of 7SP cells into which ATG-mRNA(ND3)-polyA/MITO or $tR^G$-mRNA(ND3)-$tR^R$/MITO was introduced, and $tR^G$-mRNA(ND3)-$tR^R$ or ATG-mRNA(ND3)-polyA was introduced using a commercially available nucleic acid transfection reagent.

[Figure 10] Figure 10 is a graph showing the results of measurements of mitochondrial respiration capacity of 7SP cells into which ATG-mRNA(ND3)-polyA/MITO or $tR^G$-mRNA(ND3)-$tR^R$/MITO was introduced, and $tR^G$-mRNA(ND3)-$tR^R$ or ATG-mRNA(ND3)-polyA was introduced using a commercially available nucleic acid transfection reagent.

[Figure 11] Figure 11 is a graph expressing mitochondrial respiration capacity by basal respiration (basal OCR, upper left), ATP production (ATP-linked OCR, upper right), maximal respiration (maximal OCR, lower left) and spare respiratory capacity (Spare Capacity, lower right).

[Figure 12] Figure 12 shows the results of the evaluation of cell uptake capacity when adding ATG-mRNA(ND3)-polyA/MITO to 7SP cells.

[Figure 13] Figure 13 is a fluorescence micrograph showing that ATG-mRNA(ND3)-polyA/MITO is localized in the mitochondria of the cells into which it was introduced. The left side of the figure shows a fluorescently labeled lipid membrane structure, the center of the figure shows mitochondria, and the right side of the figure shows the two superposed.

Description of Embodiments

Nucleic acid and lipid membrane structure encapsulating the same

The present invention provides a nucleic acid as shown in any of the following a) to d), and a mitochondria-targeted lipid membrane structure encapsulating the same:

a) an RNA comprising, in this order, a nucleotide sequence of a first mitochondrial tRNA, a nucleotide sequence of an mRNA encoding a target protein, and a nucleotide sequence of a second mitochondrial tRNA, wherein the nucleotide sequence of the mRNA has one or more UGAs as a tryptophan codon,
b) a DNA comprising a nucleotide sequence of a promoter and a nucleotide sequence complementary to the RNA of a),
c) an RNA comprising a nucleotide sequence of an mRNA encoding a target protein, and a nucleotide sequence of a poly(A) chain present at the 3' end side thereof, wherein the nucleotide sequence of the mRNA has one or more UGAs as a tryptophan codon, AUG as a start codon, and UAA as a stop codon,
d) a DNA comprising a nucleotide sequence of a promoter and a nucleotide sequence complementary to the RNA of c).

The RNA of a) comprises, in this order, the nucleotide sequence of a first mitochondrial tRNA (hereinafter referred to as mt-tRNA), the nucleotide sequence of an mRNA encoding a target protein, and the nucleotide sequence of a second mt-tRNA.

In the RNA of a), the nucleotide sequence of mt-tRNA can be arbitrarily selected from the nucleotide sequences of a total of 22 human mt-tRNA genes contained in the mitochondrial genome. Moreover, the nucleotide sequences of the first and second mt-tRNAs may be different from each other or may be the same.

In the present invention, the target protein is any protein that is desired to be expressed in mitochondria, in other words, to exert its function, and includes proteins that are expected to be effective in the treatment of mitochondrial diseases, as well as proteins for which there is an academic interest in being expressed in mitochondria. From such viewpoint, the target protein may be either a wild-type mitochondrial protein derived from nuclear DNA, a wild-type mitochondrial protein derived from mitochondrial DNA, an active mutant thereof, or any heterologous protein. A particularly preferable target protein is a wild-type mitochondrial protein derived from nuclear DNA or a wild-type mitochondrial protein derived from mitochondrial DNA. Moreover, the size (number of amino acid residues) and chemical properties (hydrophobicity, hydrophilicity, electric charge and other properties) of the target protein are not especially limited.

The nucleotide sequence of the mRNA encoding the target protein is any nucleotide sequence that can be finally translated into the target protein in mitochondria, and may consist of a single open reading frame (ORF) or may have a configuration in which a plurality of exons are separated by introns. In addition, it is preferable that the nucleotide sequence of the mRNA encoding the target protein have one or more UGAs as a tryptophan (Trp) codon. The Trp codon may be UGA, which is the codon corresponding to the Trp residue of the target protein, that has been artificially changed from UGG, or it may be a codon corresponding to the amino acid residue at the position where the activity of the target protein can be retained even when the amino acid residue is replaced with the Trp residue, that has been artificially changed to UGA. Such artificial modification of the codon can be performed by a general gene recombination technique.

In the translation from mRNA in the cytoplasm, UGA corresponds to a stop codon, whereas in the translation from mRNA in mitochondria, UGA corresponds to the codon encoding Trp. Thus, when animal cells are transformed using the RNA of a), the synthesis of the target protein is stopped at the UGA position even when the RNA of a) is delivered to the cytoplasmic ribosome, and the target protein is therefore not synthesized, at least not in its entirety. As a result, it is possible to suppress the negative influence caused by the synthesis of the entire target protein in the cytoplasm.

On the other hand, when the RNA of a) is delivered to mitochondria, the synthesis of the target protein proceeds without stopping at the UGA position, and as a result, the entire target protein can be properly synthesized and exert its function in the mitochondria.

From the viewpoint of suppressing undesired expression of the target protein in the cytoplasm, the use of a nucleotide sequence containing a plurality of UGAs is preferable. In addition, from the viewpoint of more reliably suppressing undesired function expression of the target protein in the cytoplasm, it is further preferable that one or more UGAs be contained at a position closer to the 5' end (N-terminus of the target protein). Furthermore, when the target protein is a protein that is usually translated in the cytoplasm, it is preferable to appropriately modify the coding sequence so that the mitochondrial codons be used for the codons other than UGA as well. One example of the above is to modify AGG, which encodes an Arg residue in the cell nuclear genome but encodes a stop codon in the mitochondrial genome, into CGG which encodes an Arg residue in both the cell nuclear genome and the mitochondrial genome.

It is preferable that the nucleotide sequence of the mRNA encoding the target protein further have AUG as a start codon and UAA as a stop codon. While AUG and UAA are respectively a major start codon and stop codon in mRNA derived from nuclear DNA, an improvement in translation efficiency can be expected by employing them in mRNA encoding target proteins to be expressed in mitochondria.

In the RNA of a), the nucleotide sequence of the first mt-tRNA, the nucleotide sequence of the mRNA encoding the target protein, and the nucleotide sequence of the second mt-tRNA may be linked via an intervening sequence, such as a flanking sequence on each 5' end side and 3' end side in the mitochondrial genome. However, it is preferable that they be linked directly without an intervening sequence, that is, that the nucleotide sequence of the mRNA and the nucleotide sequences of the two mitochondrial tRNAs be contiguous. When an intervening sequence is present, its length is about 1 to 10 nucleotides, for example 1 to 6 nucleotides, preferably 1 to 3 nucleotides. Moreover, the RNA of a) may further have about 1 to 10 nucleotides, for example 1 to 6 nucleotides, preferably 1 to 3 nucleotides of the above flanking sequence on the 5' end side of the nucleotide sequence of the first mt-tRNA and the 3' end side of the nucleotide sequence of the second mt-tRNA.

When the RNA of a) is delivered into mitochondria, the mRNA encoding the target protein, which is between the first and second mt-tRNAs, is translated, and the target protein is synthesized.

Note that, in the present invention, the RNA of a) may be RNA containing a plurality of nucleotide sequences of an mRNA encoding a target protein, and in which each of the nucleotide sequences of the mRNA has a nucleotide sequence of mitochondrial tRNA at its 5' end side and 3' end side. When such RNA contains, for example, two nucleotide sequences of an mRNA encoding a target protein, the RNA can be expressed as "an RNA comprising, in this order, a nucleotide sequence of a first mitochondrial tRNA, a nucleotide sequence of a first mRNA encoding a target protein, a nucleotide sequence of a second mitochondrial tRNA, a nucleotide sequence of a second mRNA encoding a target protein and a nucleotide sequence of a third mitochondrial tRNA, wherein each of the nucleotide sequences of the first and second mRNAs have one or more UGAs as a tryptophan codon". The details of such RNA are as described above for the RNA of a), and the nucleotide sequences of the plurality of mRNAs encoding a target protein contained therein and the nucleotide sequences of the plurality of mitochondrial tRNAs may be different from each other, or may be the same.

The DNA of b) is a DNA comprising a nucleotide sequence of a promoter and a nucleotide sequence complementary to the RNA of a), that is, the RNA of the above a). Specifically, the DNA of b) is a DNA comprising, in this order, a nucleotide sequence of a promoter, a nucleotide sequence complementary to the nucleotide sequence of the first mt-tRNA, a nucleotide sequence complementary to the nucleotide sequence of an mRNA encoding a target protein, and a nucleotide sequence complementary to the nucleotide sequence of the second mt-tRNA. Here, the respective characteristics of the nucleotide sequences of the first and second mt-tRNAs, the target protein and the nucleotide sequence of the mRNA encoding the same are as described for the RNA of a) .

The promoter sequence has a nucleotide sequence complementary to the RNA of a) under its control. Being under the control of a promoter sequence means that the RNA of a) is linked to the promoter sequence in such a way that it can transcribed, that is, that a nucleotide sequence complementary to the RNA of a) is present within the range where transcription is initiated by the transcriptional activity of the promoter sequence. An example is the case where the first mt-tRNA is located within the range of about 1 to 200 nucleotides from the 3' end of the promoter sequence. However, such a range varies depending on the type of promoter sequence and can be appropriately adjusted by those skilled in the art.

The promoter is not particularly limited as long as it exhibits transcriptional activity, that is, the ability to induce mRNA transcription. However, in order to more efficiently and selectively express a target protein when delivered to mitochondria, promoters exhibiting transcriptional activity in the cell nucleus of mammals are preferable, as described in Patent Literatures WO 2017/090763 and US 2018/0362999 (these publications are hereby incorporated by reference in their entirety). Examples of promoters exhibiting transcriptional activity in the cell nucleus of mammals include the cytomegalovirus (CMV) promoter, simian virus (SV) 40 promoter, rous sarcoma virus (RSV) promoter, EF1$\alpha$ promoter, $\beta$-actin promoter and T7 promoter, and the use of the CMV promoter or RSV promoter is preferable. In addition, these may have substitutions or other mutations on the nucleotide sequence as long as the transcriptional activity is not impaired.

The DNA of b) may be in a single-stranded form, or may be in a double-stranded form with a DNA consisting of a nucleotide sequence complementary thereto.

When the DNA of b) is delivered into mitochondria, the promoter functions to transcribe and synthesize the RNA of a) in mitochondria. The mRNA encoding the target protein is then translated, and the target protein synthesized.

The RNA of c) is an RNA comprising a nucleotide sequence of an mRNA encoding a target protein, and a nucleotide sequence of a poly(A) chain present at the 3' end side thereof, wherein the nucleotide sequence of the mRNA has one or more UGAs as a tryptophan codon, AUG as a start codon, and UAA as a stop codon.

In the RNA of c), the characteristics of the target protein and the mRNA encoding the same are as described for the RNA of a). Moreover, the poly(A) chain is a sequence of consecutive A's found downstream of the 3' untranslated region in eukaryotic mature mRNA, and its length is of about 40 to 60 bases.

In the RNA of c), the nucleotide sequence of an mRNA encoding a target protein and the nucleotide sequence of the poly(A) chain may be linked via an intervening sequence, for example, several to several tens of nucleotides of flanking sequence on each 5' end side and 3' end side in the mitochondrial genome. However, it is preferable that they be linked directly without an intervening sequence, that is, that the nucleotide sequence of an mRNA encoding the target protein and the nucleotide sequence of the poly(A) chain be contiguous. When an intervening sequence is present, its length is of about 1 to 10 nucleotides, for example 1 to 6 nucleotides, preferably 1 to 3 nucleotides. In addition, the RNA of c) may further have about 1 to 10 nucleotides, for example 1 to 6 nucleotides, preferably 1 to 3 nucleotides of the above flanking sequence on the 5' end side of the nucleotide sequence of an mRNA encoding a target protein and the 3' end side of the nucleotide sequence of the poly(A) chain.

The DNA of d) is a DNA comprising a nucleotide sequence of a promoter and a nucleotide sequence complementary to the RNA of c). Specifically, the DNA of d) is a DNA comprising, in this order, a nucleotide sequence of a promoter, a nucleotide sequence complementary to the nucleotide sequence of an mRNA encoding a target protein, and a nucleotide sequence complementary to the nucleotide sequence of the poly(A) chain.

Here, the promoter is as described for the DNA of b), the characteristics of the target protein and the nucleotide sequence of an mRNA encoding the same are as described for the RNA of a), and the characteristics of the nucleotide sequence of the poly(A) chain are as described for the RNA of c). In addition, the promoter sequence has a nucleotide sequence complementary to the RNA of c) under its control, and its significance is as described for the DNA of b).

When the DNA of d) is delivered into mitochondria, the promoter functions to transcribe and synthesize the RNA of c) in mitochondria, and the target protein is synthesized after translation of the mRNA.

A first aspect of the present invention relates to a mitochondria-targeted lipid membrane structure encapsulating the nucleic acid of any of the above a) to d). The mitochondria-targeted lipid membrane structure in the first aspect contains sphingomyelin (SM), preferably dioleylphosphatidylethanolamine (DOPE) and SM as constituent lipids of the lipid membrane. It is also preferable that the mitochondria-targeted lipid membrane structure have a peptide consisting of the amino acid sequence set forth in SEQ ID NO: 1 (hereinafter referred to as KALA peptide) on the lipid membrane surface.

A preferred specific example of the mitochondria-targeted lipid membrane structure is the lipid membrane structure containing DOPE and SM as constituent lipids of the lipid membrane, and in which the lipid membrane surface has been modified with an octaarginine peptide, as described in detail together with the production method thereof in Japanese Patent No. 5067733 and WO 2006/095837, or the lipid membrane structure containing DOPE and SM as constituent lipids of the lipid membrane, and in which the lipid membrane surface has been modified with a KALA peptide, as described in detail together with the production method thereof in WO 2017/090763 and US 2018/0362999. These publications are hereby incorporated by reference in their entirety.

The KALA peptide is described by Shaheen et al. (Biomaterials, 2011, 32: 6342-6350) as a membrane-fusogenic peptide having the function of promoting membrane fusion between lipid membranes. In a preferred example of the above lipid membrane structure, the ability to selectively deliver the lipid membrane structure to the intracellular organelle mitochondria by having the KALA peptide placed on the surface of the lipid membrane structure is utilized.

The most preferable lipid membrane structure in the first aspect of the present invention is a lipid membrane structure encapsulating the nucleic acid of any of a) to d) above, containing DOPE and SM as constituent lipids of the lipid membrane, and having a KALA peptide on the surface of the lipid membrane. This lipid membrane structure can be produced by substituting DNA by any of the nucleic acids of a) to d) above according to the method for producing a lipid membrane structure modified with a KALA peptide and encapsulating DNA described in WO 2017/090763 and US 2018/0362999.

Pharmaceutical composition

The present invention provides, as another aspect, a pharmaceutical composition for treating and/or preventing mitochondrial diseases, which comprises as an active ingredient the nucleic acid represented by any of a) to d) having the characteristics as described in the first aspect. Such pharmaceutical composition may directly comprise the nucleic acid represented by any of a) to d) having the characteristics as described in the first aspect. However, it is particularly preferably a pharmaceutical composition comprising as an active ingredient a nucleic acid in the form of the mitochondria-targeted lipid membrane structure of the first aspect.

The pharmaceutical composition of the present aspect can be used in the form of a parenteral preparation such as an injection or a drip. Examples of carriers that can be used for such parenteral preparations include aqueous carriers such as physiological saline and isotonic solutions containing glucose, D-sorbitol and the like.

The pharmaceutical composition of the present aspect may contain components such as pharmaceutically acceptable buffers, stabilizers, preservatives and other additives. The pharmaceutically acceptable components are well known to those skilled in the art, who can appropriately select and use them, for example, from the components described in the Japanese Pharmacopoeia 17th Edition and other standards, depending on the form of the preparation and within the range of normal implementation capacity.

The method for administering the pharmaceutical composition of the present aspect is not particularly limited, but in the case of a parenteral preparation, examples thereof include intravascular administration (preferably intravenous administration), intraperitoneal administration, intestinal administration, and subcutaneous administration. In one preferred embodiment, the therapeutic agent of the present invention is administered to a living body by intravenous administration.

Mitochondrial diseases are one of the intractable diseases that were recognized in 2009 as a subject of the Specific Diseases Treatment Research Program, which is one of the measures to prevent intractable diseases in Japan, and are a general term for pathological conditions in which mitochondrial function is impaired and clinical symptoms appear. At present, a decrease in energy production is considered to be the main cause of dysfunction in mitochondrial diseases. The etiology of mitochondrial diseases can be divided into abnormalities in the nuclear genomic DNA and abnormalities in the mitochondrial genomic DNA (mt-DNA). The latter includes deletions/duplications of bases on mt-DNA, point mutations (qualitative changes) and a decrease (quantitative change) in the amount of mt-DNA.

The pharmaceutical composition of the present aspect is expected to exhibit a therapeutic and/or preventive effect on mitochondrial diseases by supplying to mitochondria an mRNA encoding a wild-type protein corresponding to the individual gene abnormalities causing the mitochondrial diseases.

Treatment and/or prevention as used in the present description includes all types of medically acceptable therapeutic and/or preventive interventions intended for the cure, temporary remission, prevention and the like of a disease. That is, the treatment and/or prevention of mitochondrial diseases includes medically acceptable interventions for various purposes, including delaying or stopping the progress of mitochondrial diseases, the regression or disappearance of lesions, and the prevention of onset or prevention of recurrence.

The pharmaceutical composition of the present aspect can be used for treatment and/or prevention of mitochondrial diseases. Therefore, the use of the pharmaceutical composition of the present aspect can also be expressed as a method for treating and/or preventing mitochondrial diseases using the composition, and the present invention also provides an invention of such a method.

Furthermore, administering the pharmaceutical composition of the present aspect to a patient with a mitochondrial disease or a person at risk of developing a mitochondrial disease is expected to allow the treatment and/or prevention of the mitochondrial disease. Thus, the present invention also provides a method for treating and/or preventing mitochondrial diseases by administering an effective amount of the pharmaceutical composition of the present aspect to a patient with a mitochondrial disease or a person at risk of developing a mitochondrial disease. Here, the "effective amount" means an amount effective for treating and/or preventing a mitochondrial disease, and such an amount is appropriately adjusted depending on the degree of symptoms of the mitochondrial disease and other medical factors.

## Method for producing cell preparation

The present invention provides, as a further aspect, a method for producing a cell preparation for treating and/or preventing mitochondrial diseases, the method comprising introducing *in vitro* the nucleic acid represented by any of a) to d) having the characteristics as described in the first aspect, into cells derived from a patient with a mitochondrial disease or a person at risk of developing a mitochondrial disease.

It is preferable that the cells derived from a patient with a mitochondrial disease or a person at risk of developing a mitochondrial disease, into which the nucleic acid is to be introduced, be somatic cells collected from a patient with a mitochondrial disease or a person at risk of developing a mitochondrial disease. Examples of such somatic cells include cells that have been separated from the heart, brain, skeletal muscle of these persons, or other tissues damaged or at risk of being damaged by the mitochondrial disease, or cells having the ability to differentiate into cells that constitute such tissues.

The nucleic acid to be introduced into the cells derived from a patient with a mitochondrial disease or a person at risk of developing a mitochondrial disease may be in its original form, but from the viewpoint of introduction efficiency, it is preferably in the form of the mitochondria-targeted lipid membrane structure, which is the first aspect of the present invention.

By introducing *in vitro* the nucleic acid represented by any of a) to d) having the characteristics as described in the first aspect into cells derived from a patient with a mitochondrial disease or a person at risk of developing a mitochondrial disease, it is possible to produce somatic cells with a reduced mutation rate in the mitochondrial mRNA (mt-mRNA) encoding the mitochondrial protein causing the mitochondrial disease. These cells can be returned to the patient with a mitochondrial disease and used as a cell preparation for treating and/or preventing the mitochondrial disease.

In the present invention, the mutation rate in mt-mRNA means the proportion of mutant molecules in the total number of mt-mRNA molecules for a specific mt-mRNA encoding a mitochondrial protein causing a mitochondrial disease.

The mutation rate in mt-mRNA can be quantified using a known method that can be used for quantifying gene mutations, such as the allele-specific PCR method, the invader method, the allele-specific hybridization method, and the next-generation sequencing method. Of these methods, one method preferably used is the quantitative ARMS-PCR method (Amplification Refractory Mutation Systems PCR; for example, V. Venegas et al, Methods Mol. Biol. 2012, 837, 313-326; this publication is hereby incorporated by reference in its entirety). In this method, a primer DNA set for amplifying a wild-type sequence (wild-type detection primer DNA set) and a primer DNA set for amplifying a sequence containing a point mutation (mutant detection primer DNA set) are designed based on the base of the point mutation to be detected and the nucleotide sequences preceding and following it. Then, quantitative PCR using as a template the DNA obtained by reverse transcription reaction of the mt-mRNA to be measured is performed using each primer DNA set. The mutation rate can be calculated by applying the $C_T$ value obtained when using the wild-type detection primer DNA set ($C_{Twild}$) and the $C_T$ value obtained when using the mutant detection primer DNA set ($C_{Tmut}$) determined by quantitative PCR to the following formula:

[Expression 1]

$$\text{Mutation rate (\%)} = \frac{1}{1+(\frac{1}{2})^{\Delta C^{T}}} \times 100$$

$$\Delta C_T = C_{Twild} - C_{Tmut}$$

<u>Primer DNA for detecting point mutation</u>

The present invention further provides a kit for detecting T10158C, which is a point mutation of the mitochondrial ND3 protein, the kit comprising a wild-type detection primer DNA set consisting of a combination of a primer DNA containing the nucleotide sequence set forth in SEQ ID NO: 2 and a primer DNA consisting of the nucleotide sequence set forth in SEQ ID NO: 4, and a mutant detection primer DNA set consisting of a combination of a primer DNA containing the nucleotide sequence set forth in SEQ ID NO: 2 and a primer DNA consisting of the nucleotide sequence set forth in SEQ ID NO: 10.

The point mutation T10158C is a gene mutation that is one of the causes of mitochondrial disease (McFarland R. et al., Ann Neurol. 2004, 55(1):58-64, and the like), in which the nucleotide at position 10158 in the nucleotide sequence of the mt-DNA (nucleotide sequence of human mitochondrial genomic DNA, NCBI Reference Sequence: NC_012920.1) that is thymine in normal cases, has been replaced by cytosine.

The wild-type detection primer DNA set consisting of the combination of a primer DNA containing the nucleotide sequence set forth in SEQ ID NO: 2 and a primer DNA consisting of the nucleotide sequence set forth in SEQ ID NO: 4 can specifically detect mt-DNA in which the nucleotide at position 10158 is thymine or the mt-mRNA corresponding thereto, by performing PCR using cDNA, which is the reverse transcription reaction product from the mt-DNA or mt-mRNA to be detected, as a template.

Moreover, the mutant detection primer DNA set consisting of the combination of a primer DNA containing the nucleotide sequence set forth in SEQ ID NO: 2 and a primer DNA consisting of the nucleotide sequence set forth in SEQ ID NO: 10 can specifically detect mt-DNA in which the 10158th base is cytosine or the mt-mRNA corresponding thereto, by performing PCR using DNA, which is the reverse transcription reaction product from the mt-DNA or mt-mRNA to be detected, as a template.

The primer DNA containing the nucleotide sequence set forth in SEQ ID NO: 2 can be, for example, a primer DNA consisting of the nucleotide sequence set forth in SEQ ID NO: 3, a primer DNA consisting of a nucleotide sequence to which one or more nucleotides, for example 1 to 10, preferably 1 to 5, more preferably 1 to 3, particularly 1 or 2 nucleotides were added to the 5' end side and/or 3' end side of the nucleotide sequence set forth in SEQ ID NO: 3, or a primer DNA consisting of a nucleotide sequence in which one or more nucleotides, for example 1 to 5, preferably 1 to 3, particularly 1 or 2 nucleotides at the 5' end and/or the 3' end have been deleted in the nucleotide sequence set forth in SEQ ID NO: 3. A preferred example of the primer DNA consisting of a nucleotide sequence in which one or more nucleotides at the 5' end and/or the 3' end have been deleted in the nucleotide sequence set forth in SEQ ID NO: 3 is the primer DNA consisting of the nucleotide sequence set forth in SEQ ID NO: 2.

Using the wild-type detection primer DNA set and the mutant detection primer DNA set, the ratio of molecules having the point mutation T10158C contained in the mt-DNA or cDNA, that is, the mutation rate, can be quantified by performing PCR using cDNA, which is the reverse transcription reaction product from mt-DNA or mt-mRNA as a template. The details of the mutation rate quantification are as described above.

The kit of the present aspect is any kit containing a wild-type detection primer DNA set and a mutant detection primer DNA set, but may further contain a reverse transcriptase, reagents and buffers for PCR reaction, and the like.

The present invention is further described in detail with the following examples, but the present invention is not limited to these examples.

Examples

Example 1: Production of liposome encapsulating mRNA

1) Production of DNA for mRNA preparation

A recombinant DNA cassette ($tR^G$-mRNA(ND3)-$tR^R$, SEQ ID NO: 11, Figure 1) having, in this order, a T7 promoter, the nucleotide sequence corresponding to the mitochondrial $tRNA^{Gly}$ containing 20 bases on the 5' end side, an ORF encoding wild-type ND3 protein, and the nucleotide sequence corresponding to mitochondrial $tRNA^{Arg}$ containing 24 bases on the 3' end side, was synthesized. In addition, a recombinant DNA cassette (ATG-mRNA(ND3)-polyA, SEQ ID NO: 12, Figure 2) having an ORF encoding a mitochondrial ND3 protein in which the start codon was replaced from ATA to ATG, the T corresponding to the stop codon was replaced with TAA, and a poly(A) sequence of 50 bases was added to the 5' end, was synthesized. Each DNA cassette was incorporated into the plasmid pUC57-Amp opened with the restriction enzymes EcoRI and EcoRV to prepare two plasmid DNAs (pT7-$tR^G$-mRNA(ND3)-$tR^R$ and pT7-ATG-mRNA(ND3)-polyA) in which mRNA is transcribed from each cassette by the T7 promoter.

2) mRNA Preparation

The plasmid DNA prepared in 1) was treated with the restriction enzyme EcoRV to prepare a linearized plasmid DNA, which was then subjected to an *in vitro* transcription reaction using RiboMAX (trademark) Large Scale RNA Production Systems-T7 (PROMEGA) to synthesize RNA (SEQ ID NOS: 13 and 14), transcribed by the T7 promoter, followed by purification using RNA clean-up and concentration of RNA (NORGEN).

3) Preparation of MITO-Porter encapsulating mRNA

To 0.18 mg/mL of protamine/HEPES Buffer solution, an equal volume of 0.3 mg/mL of purified mRNA/HEPES Buffer solution was added while vortexing to prepare a nucleic acid nanoparticle solution (N/P ratio 0.9). 65 μL of the nucleic acid nanoparticle solution was added to a lipid solution (DOPE:SM:STR-R8 = 9:2:1, 18 μL DOPE, 8 μL SM, 20 μL STR-R8, 62 μL EtOH) while vortexing to prepare a suspension. The suspension was added to 630 μL of HEPES Buffer in a 5 mL tube while vortexing, then the mixture was added to 4 mL of HEPES Buffer in Amicon Ultra-4, and centrifuged (1000 g, 25°C, 8 min). 4 mL of HEPES Buffer was further added, and the mixture was centrifuged again (1000 g, 25°C, 12 min) to collect the liposomes encapsulating mRNA. The liposome encapsulating mRNA transcribed from pT7-$tR^G$-mRNA(ND3)-$tR^R$ is represented as $tR^G$-mRNA(ND3)-$tR^R$/MITO and the liposome encapsulating mRNA transcribed from pT7-ATG-mRNA(ND3)-polyA is represented as ATG-mRNA(ND3)-polyA/MITO. The particle diameter and zeta potential (surface potential) of each liposome is shown in Table 1.

[Table 1]

| | Particle diameter (nm) | | PdI | Zeta potential (mV) |
|---|---|---|---|---|
| $tR^G$-mRNA(ND3)-$tR^R$/MITO | Core | 146 | 0.01 | -38 |
| | Liposome | 155 | 0.256 | 41 |
| ATG-mRNA(ND3)-polyA/MITO | Core | 165 | 0.101 | -38 |
| | Liposome | 175 | 0.286 | 42 |

Example 2. Establishment of protocol for quantifying point mutation (T10158C) in mitochondrial RNA

1) Primer design

Primer DNAs consisting of the nucleotide sequences shown in Table 2 were chemically synthesized.

[Table 2]

| | | | |
|---|---|---|---|
| Common forward primer | F | CAACACCCTCCTAGCCTTAC | SEQ ID NO: 2 |
| | F long | ATCAACACCCTCCTAGCCTTACTA | SEQ ID NO: 3 |
| Wild-type detection reverse primer | WT 1 | CCGCACTCGTAAGGGGTGCA | SEQ ID NO:4 |
| | WT2 | CCGCACTCGTAAGGGGTCCA | SEQ ID NO: 5 |
| Mutant detection reverse primer | MT1 | CCGCACTCGTAAGGGGTGCG | SEQ ID NO: 6 |
| | MT2 | CCGCACTCGTAAGGGGTCCG | SEQ ID NO: 7 |
| | MTO | CCGCACTCGTAAGGGGTGGG | SEQ ID NO: 8 |
| | MTO long | AGCCGCACTCGTAAGGGGTGGG | SEQ ID NO: 9 |
| | MT1 long | AGCCGCACTCGTAAGGGGTGCG | SEQ ID NO: 10 |

A base substitution corresponding to the point mutation (T10158C) was introduced into the plasmid DNA containing the ORF encoding the wild-type ND3 protein produced in Example 1 (pT7-ATG-mRNA(ND3)-polyA) to produce a plasmid DNA having the ORF corresponding to the point mutation (T10158C) of ND3 (pT7-ATG-mRNA(ND3)MT-polyA).

2) Calibration curve preparation

Quantitative PCR was performed using the primer DNA sets 1 to 11 consisting of the combinations shown in Table 3 and SYBR Green Realtime PCR Master Mix (TOYOBO), using as a template a mixed DNA solution in which pT7-ATG-mRNA(ND3)-polyA and pT7-ATG-mRNA(ND3)MT-polyA were mixed at a predetermined ratio. The PCR conditions were 95°C/1 min -> {95°C/15 sec -> 60°C/1 min} for 40 cycles.

[Table 3]

Set **1**: $C_{Twild\ type}$ : Forward (F) x Reverse (WT1) / $C_{Tmut\ type}$ : Forward (F) x Reverse (MT1)
Set **2**: $C_{Twild\ type}$ : Forward (F) x Reverse (WT1) / $C_{Tmut\ type}$: Forward (F) x Reverse (MT2)
Set **3**: $C_{Twild\ type}$ : Forward (F) x Reverse (WT2) / $C_{Tmut\ type}$ : Forward (F) x Reverse (MT1)
Set **4**: $C_{Twild\ type}$ : Forward (F) x Reverse (WT2) / $C_{Tmut\ type}$: Forward (F) x Reverse (MT2)
Set **5**: $C_{Twild\ type}$ : Forward (F) x Reverse (WT1)/ $c_{Tmut\ type}$ : Forward (F) x Reverse (MTO)
Set **6**: $C_{Twild\ type}$ : Forward (F) x Reverse (WT1) / $C_{Tmut\ type}$ : Forward (F) x Reverse (MT0 long)
Set **7**: $C_{Twild\ type}$ : Forward (F) x Reverse (WT1)/$C_{Tmut\ type}$ : Forward (F) x Reverse (MT1 long)
Set **8**: $C_{Twild\ type}$ : Forward (F long) x Reverse (WT1) / $C_{Tmut\ type}$: Forward (F long) x Reverse (MTO)
Set **9**: $C_{Twild\ type}$ : Forward (F long) x Reverse (WT1)/ $C_{Tmut\ type}$: Forward (F long) x Reverse (MTO long)
Set **10**: $C_{Twild\ type}$ : Forward (F long) x Reverse (WT1)/ $C_{Tmut\ type}$ : Forward (F long) x Reverse (MT1)
Set **11**: $C_{Twild\ type}$ : Forward (F long) x Reverse (WT1)/ $C_{Tmut\ type}$ : Forward (F long) x Reverse (MT1 long)

The $C_T$ values obtained by PCR using the primer DNA set containing the wild-type detection reverse primer ($C_{Twild}$) and the $C_T$ values obtained by PCR using the primer DNA set containing the mutant detection reverse primer ($C_{Tmut}$) were applied to the following formula to calculate the mutation rate in each mixed DNA solution.

[Expression 2]

$$\text{Mutation rate (\%)} = \frac{1}{1+(\frac{1}{2})^{\Delta C^{T}}} \times 100$$

$$\Delta C_T = C_{Twild} - C_{Tmut}$$

A calibration curve was prepared by plotting the theoretical values of the mutation rate (mixture ratio of normal DNA and mutant DNA) on the horizontal axis and the mutation rates calculated from the $C_T$ values on the vertical axis (Figure 3). As a result, it was confirmed that primer DNA set 7 (F: SEQ ID NO: 2 and R: SEQ ID NO: 4 for the wild-type detection set, and F: SEQ ID NO: 2 and R: SEQ ID NO: 10 for the mutant detection set) and primer DNA set 11 (F: SEQ ID NO: 3 and R: SEQ ID NO: 4 for the wild-type detection set, and F: SEQ ID NO: 3 and R: SEQ ID NO: 10 for the mutant detection set) give a linear calibration curve with a slope of nearly 1. When the test was performed three times on these two primer DNA sets, a calibration curve with high reproducibility was obtained for both sets, which confirmed that these sets are suitable for quantifying the T10158C mutation rate (Figure 4).

Example 3. Quantification of T10158C mutation rate in 7SP cells

1) Preparation of cells derived from patient with mitochondrial disease

Skin fibroblast (7SP) cells from a skin biopsy of a patient with a mitochondrial disease (Leigh syndrome) having a point mutation (T10158C) in the ND3 gene were separated and subcultured, then cryopreserved to prepare a cell stock.

1 mL of the cell stock was dissolved in a 37°C water bath, to which 9 mL of DMEM (FBS+) was added, and the mixture was centrifuged (100 g, 4°C, 7 min). The supernatant was removed, and 10 mL of DMEM (FBS+) was added to suspend the cells. The total volume was then transferred to a 10 cm dish, and cultured by incubation (37°C, 5% $CO_2$). The cells were passaged when the confluency was reached at 80-90%. After washing the dish with 5 mL of PBS(-), a 2 mL of 0.25% trypsin solution was added, and the mixture was incubated (37°C, 5% $CO_2$) for 2 to 3 min. 8 mL of DMEM (FBS+) was then added, and the mixture was centrifuged (100 g, 4°C, 7 min). After counting, the cells were seeded in a 10 cm dish at an appropriate concentration.

2) Preparation of mitochondrial fraction and DNA for quantification

7SP cells (about $1 \times 10^6$ cells) were suspended in 100 μL of Cell Scrub buffer, shaken at 4°C for 15 min, and then centrifuged (700 g, 4°C, 3 min). After removing the supernatant, the cells were suspended in 500 μL of MIB (EDTA+). After disrupting the cells with a syringe needle (27G), centrifuging (700 g, 4°C, 10 min) and collecting the supernatant, 0.5 μL of 0.1 mg/mL RNase solution was added and the mixture was incubated at 25°C for 10 min. The mixture was centrifuged (700 g, 4°C, 10 min), and the supernatant was collected and added to a 60% percoll solution. The mixture was centrifuged (20,400 g, 4°C, 10 min), and 200 μL of the solution on the boundary surface was collected. After centrifuging (20,400 g, 4°C, 15 min) and removing the supernatant, 500 μL of MIB (-) was added. The mixture was again centrifuged (20,400 g, 4°C, 15 min) and the supernatant removed to obtain the mitochondrial fraction.

RNA was extracted from the mitochondrial fraction using an RNase mini kit (QIAGEN NV) and an RNase-Free DNAse Set (QIAGEN NV), and a reverse transcription reaction of the RNA was further performed using the High Capacity RNA-to-cDNA Kit (ABI) to prepare a cDNA for quantification.

3) Quantification of mutation rate

The mutation rate of T10158C in cDNA complementary to mitochondrial ND3 mRNA of 7SP cells was 82.1% when quantified using the primer DNA set 7 of Example 2. Since the mutation rate of HeLa cells without T10158C was 0.86%, substantially 0%, when quantified in the same manner, the T10158C quantification system using the primer DNA set 7 was considered to be functioning normally.

Example 4. Introduction of normal mRNA into cells derived from patient with mitochondrial disease

ATG-mRNA(ND3)-polyA/MITO was added to 7SP cells ($1 \times 10^5$ cells/well) pre-cultured with 2 mL/well of DMEM (FBS+) for about 24 hours on a 6-well plate, so as to be 0.6, 3, 6, 30, and 60 ng/well in terms of RNA amount, and the mixtures were incubated for 3 hours. Then, the culture media were exchanged and the mixtures incubated for another 48 hours. After the incubation, the same operation as in Example 3 was performed to prepare cDNA for quantification from the mitochondrial fraction of the 7SP cells, and quantitative PCR using the primer DNA set 7 was performed to measure the changes in T10158C mutation rate in the cDNA complementary to ND3 mRNA. As a result, it was confirmed that the mutation rate decreased depending on the amount of RNA added, and that the addition of 60 ng of RNA decreased the mutation rate to 2% (Figure 5, top). Even when the incubation time after the culture medium exchange was 72 hours, a similar decrease in the mutation rate depending on the amount of RNA added was observed (Figure 5, bottom). This tendency was also observed when $tR^G$-mRNA(ND3)-$tR^R$/MITO was used instead of ATG-mRNA(ND3)-polyA/MITO.

Example 5: Introduction of normal mRNA into cells derived from patient with mitochondrial disease

(1) Based on Example 4, ATG-mRNA(ND3)-polyA/MITO was added to 7SP cells so as to be 60 ng/well in terms of RNA amount, and the mixture was incubated for 3 hours. Then, the culture medium was exchanged, and the incubation was continued for 21 days. On day 2, 3, 4, 5, 7, 14 and 21 after the culture medium exchange, a part of the cultured cells was collected, and a cDNA for quantification was prepared from the mitochondrial fraction in the same manner as in Example 4 to measure the changes over time in the T10158C mutation rate in the cDNA complementary to ND3 mRNA. As a result, it was confirmed that the mutation rate was lower compared to the control (untreated cells, NT) until day 5 (Figure 6).

(2) mRNA (ATG-mRNA(ND3)-polyA) transcribed from pT7-ATG-mRNA(ND3)-polyA was introduced into 7SP cells so as to be 60 ng/well in terms of RNA amount using Lipofectamine i MAX (LFN iMAX, ThermoFisher Scientific), which is a commercially available nucleic acid transfection reagent, and the changes over time in the mutation rate up to day 7 were measured in the same manner as in (1). The results of these measurements, together with the results of the change over time from day 3 to 21 measured in (1) are shown in Figure 7. It was confirmed that the mutation rate is lower when the mRNA is introduced using the lipid membrane structure of the present invention than when the mRNA is introduced using a commercially available nucleic acid transfection reagent.

(3) In the same manner as in (1), ATG-mRNA(ND3)-polyA/MITO (ATG/MITO) and $tR^G$-mRNA(ND3)-$tR^R$/MITO (TR/MITO) were each introduced into 7SP cells so as to be 30 ng/well or 60 ng/well in terms of RNA amount, and mRNA ($tR^G$-mRNA(ND3)-$tR^R$) transcribed from ATG-mRNA(ND3)-polyA and pT7-$tR^G$-mRNA(ND3)-$tR^R$ using LFN i MAX was introduced into 7SP cells. The mutation rate of each cell after three days was then measured. The results are shown in Figure 8. Similarly to the results of (2), it was confirmed that the mutation rate is lower when the mRNA is introduced using the lipid membrane structure of the present invention than when the mRNA is introduced using a commercially available nucleic acid transfection reagent.

Example 6: Confirmation of effect of improving mitochondrial function

7SP cells were seeded on a 6-well plate at $1 \times 10^5$ cells/well (2 mL/well of a $0.5 \times 10^5$ cells/mL solution) and cultured for 24 $\pm$ 3 hours. An ATG-mRNA(ND3)-polyA/MITO or $tR^G$-mRNA(ND3)-$tR^R$/MITO solution was diluted with DMEM (FBS-) to 60 ng/well in terms of RNA amount, of which 1 mL was added to 7SP cells. After incubation for 3 hours, the culture medium was exchanged with DMEM (FBS+) and the incubation was further continued. In addition, $tR^G$-mRNA(ND3)-$tR^R$ or ATG-mRNA(ND3)-polyA (RNA amount: 60 ng/well) was introduced using LFN i MAX into 7SP cells, which were incubated for 3 hours. Then, the culture medium was exchanged with DMEM (FBS+) and the incubation was further continued.

48 hours after the culture medium exchange, oligomycin (respiratory chain complex V inhibitor; final concentration: 2.0 $\mu$M), carbonyl cyanide-p-trifluoromethoxyphenylhydrazone (FCCP, uncoupling agent; final concentration: 2.0 $\mu$M), rotenone and antimycin A (inhibitor of respiratory chain complex I and III; final concentration: 0.5 $\mu$M) were sequentially added according to the schedule shown in Figure 9, and mitochondrial oxygen consumption (OCR) was continuously measured using Seahorse XFe96 Analyzer (Agilent). Each OCR before addition of oligomycin, after addition of oligomycin, after addition of FCCP, and after addition of rotenone and antimycin A represents the basal respiration (basal OCR), ATP production (ATP-linked OCR), maximal respiration (maximal OCR), and spare respiratory capacity (Spare Capacity), respectively, among the evaluation items of mitochondrial respiration capacity. The changes in OCR over time are shown in Figure 10, and the OCR for each of the evaluation item is shown in Figure 11. In Figure 11, Mito1 represents cells introduced with ATG-mRNA(ND3)-polyA/MITO, Mito2 represents cells introduced with $tR^G$-mRNA(ND3)-$tR^R$/MITO, Mito3 represents cells introduced with the empty lipid membrane structure MITO-Porter containing no mRNA, Mito5 represents cells cell introduced with ATG-mRNA(ND3)-polyA using LFN i MAX, and Mito6 represents cells introduced with $tR^G$-mRNA(ND3)-$tR^R$ using LFN i MAX.

For both ATG-mRNA(ND3)-polyA/MITO and $tR^G$-mRNA(ND3)-$tR^R$/MITO, it was confirmed that the mitochondrial respiration capacity was higher compared to an empty lipid membrane structure without carrying mRNA and to when mRNA is introduced with a commercially available nucleic acid transfection reagent.

Example 7. Confirmation of mitochondrial localization of ATG-mRNA(ND3)-polyA/MITO

A fluorescently labeled ATG-mRNA(ND3)-polyA/MITO (F-mRNA/MITO) containing DOPE labeled with NBD (7-nitrobenz-2-oxa-1,3-diazole, green fluorescent dye) in the lipid membrane and an NBD-labeled lipid membrane structure containing no mRNA (F/MITO) were produced according to 3) of Example 1. F-mRNA/MITO and F/MITO were each added to 7SP cells ($1 \times 10^5$ cells/well) pre-cultured with 2 mL/well of DMEM (FBS+) for about 24 hours on a 6-well plate, so as to be 60 ng/well in terms of RNA amount, and the mixtures were incubated for one hour. The results of detecting the fluorescence taken up by the cells using FACS confirmed that both F-mRNA/MITO and F/MITO were taken up by the 7SP cells, and that there was no change in the amount taken up by the cells even when carrying mRNA (Figure 12).

The mitochondria of the 7SP cells taking up F-mRNA/MITO were stained red according to the method of Abe et al. (J. Pharm. Sci. 2016, 105, 734-740), and then the localization of F-mRNA/MITO in the cells was observed using a confocal laser scanning microscope. As a result, a yellow color obtained by the overlap of green (F-mRNA/MITO) and red (mitochondria) was observed, confirming that F-mRNA/MITO was localized in the mitochondria of 7SP cells (Figure 13).

Sequence Listing Free Text

SEQ ID NO: 1: KALA peptide
SEQ ID NO: 2: forward primer F
SEQ ID NO: 3: forward primer F long
SEQ ID NO: 4: reverse primer WT1
SEQ ID NO: 5: reverse primer WT2
SEQ ID NO: 6: reverse primer MT1
SEQ ID NO: 7: reverse primer MT2
SEQ ID NO: 8: reverse primer MT0
SEQ ID NO: 9: reverse primer MT0 long
SEQ ID NO: 10: reverse primer MT1 long
SEQ ID NO: 11: $tR^G$-mRNA(ND3)-$tR^R$ DNA cassette
SEQ ID NO: 12: ATG-mRNA(ND3)-polyA DNA cassette
SEQ ID NO: 13: $tR^G$-mRNA(ND3)-$tR^R$ RNA
SEQ ID NO: 14: ATG-mRNA(ND3)-polyA RNA

## Claims

1. A mitochondria-targeted lipid membrane structure encapsulating a nucleic acid represented by any of the following a) to d):

   a) an RNA comprising, in this order, a nucleotide sequence of a first mitochondrial tRNA, a nucleotide sequence of an mRNA encoding a target protein, and a nucleotide sequence of a second mitochondrial tRNA, wherein the nucleotide sequence of the mRNA has one or more UGAs as a tryptophan codon;
   b) a DNA comprising a nucleotide sequence of a promoter and a nucleotide sequence complementary to the RNA of a);
   c) an RNA comprising a nucleotide sequence of an mRNA encoding a target protein, and a nucleotide sequence of a poly(A) chain present at the 3' end side thereof, wherein the nucleotide sequence of the mRNA has one or more UGAs as a tryptophan codon, AUG as a start codon, and UAA as a stop codon; and
   d) a DNA comprising a nucleotide sequence of a promoter and a nucleotide sequence complementary to the RNA of c).

2. The lipid membrane structure according to claim 1, wherein the nucleotide sequence of the mRNA and the nucleotide sequences of the two mitochondrial tRNAs of a) are contiguous.

3. The lipid membrane structure according to claim 1, wherein the nucleotide sequence of the mRNA and the nucleotide sequence of the poly(A) chain of c) are contiguous.

4. The lipid membrane structure according to any one of claims 1 to 3, wherein the target protein is a wild-type mitochondrial protein derived from mitochondrial DNA.

5. The lipid membrane structure according to any one of claims 1 to 4, comprising dioleylphosphatidylethanolamine and sphingomyelin as constituent lipids of the lipid membrane.

6. The lipid membrane structure according to any one of claims 1 to 5, which has a peptide consisting of the amino acid sequence set forth in SEQ ID NO: 1 on the lipid membrane surface.

7. A pharmaceutical composition for treating and/or preventing mitochondrial diseases, the composition comprising as an active ingredient a nucleic acid represented by any of the following a) to d):

   a) an RNA comprising, in this order, a nucleotide sequence of a first mitochondrial tRNA, a nucleotide sequence of an mRNA encoding a target protein, and a nucleotide sequence of a second mitochondrial tRNA, wherein the nucleotide sequence of the mRNA has one or more UGAs as a tryptophan codon;
   b) a DNA comprising a nucleotide sequence of a promoter and a nucleotide sequence complementary to the RNA of a);
   c) an RNA comprising a nucleotide sequence of an mRNA encoding a target protein, and a nucleotide sequence of a poly(A) chain present at the 3' end side thereof, wherein the nucleotide sequence of the mRNA has one or more UGAs as a tryptophan codon, AUG as a start codon, and UAA as a stop codon; and
   d) a DNA comprising a nucleotide sequence of a promoter and a nucleotide sequence complementary to the RNA of c).

8. The pharmaceutical composition according to claim 7, wherein the nucleotide sequence of the mRNA and the nucleotide sequences of the two mitochondrial tRNAs of a) are contiguous.

9. The pharmaceutical composition according to claim 7, wherein the nucleotide sequence of the mRNA and the nucleotide sequence of the poly(A) chain of c) are contiguous.

10. The pharmaceutical composition according to any one of claims 7 to 9, wherein the target protein is a wild-type mitochondrial protein derived from mitochondrial DNA.

11. The pharmaceutical composition according to any one of claims 7 to 10, wherein the nucleic acid is encapsulated in a mitochondria-targeted lipid membrane structure.

**12.** A method for producing a cell preparation for treating and/or preventing mitochondrial diseases, the method comprising introducing *in vitro* the nucleic acid represented by any of the following a) to d) into cells derived from a patient with a mitochondrial disease or a person at risk of developing a mitochondrial disease:

a) an RNA comprising, in this order, a nucleotide sequence of a first mitochondrial tRNA, a nucleotide sequence of an mRNA encoding a target protein, and a nucleotide sequence of a second mitochondrial tRNA, wherein the nucleotide sequence of the mRNA has one or more UGAs as a tryptophan codon;
b) a DNA comprising a nucleotide sequence of a promoter and a nucleotide sequence complementary to the RNA of a);
c) an RNA comprising a nucleotide sequence of an mRNA encoding a target protein, and a nucleotide sequence of a poly(A) chain present at the 3' end side thereof, wherein the nucleotide sequence of the mRNA has one or more UGAs as a tryptophan codon, AUG as a start codon, and UAA as a stop codon; and
d) a DNA comprising a nucleotide sequence of a promoter and a nucleotide sequence complementary to the RNA of c).

**13.** The production method according to claim 12, wherein the nucleotide sequence of the mRNA and the nucleotide sequences of the two mitochondrial tRNAs of a) are contiguous.

**14.** The production method according to claim 12, wherein the nucleotide sequence of the mRNA and the nucleotide sequence of the poly(A) chain of c) are contiguous.

**15.** The production method according to any one of claims 12 to 14, wherein the target protein is a wild-type mitochondrial protein derived from mitochondrial DNA.

**16.** The production method according to any one of claims 12 to 15, wherein the nucleic acid is encapsulated in a mitochondria-targeted lipid membrane structure.

**17.** An RNA comprising a nucleotide sequence of an mRNA encoding a target protein, and a nucleotide sequence of a poly(A) chain present at the 3' end side thereof, wherein the nucleotide sequence of the mRNA has one or more UGAs as a tryptophan codon, AUG as a start codon, and UAA as a stop codon.

**18.** The RNA according to claim 17, wherein the nucleotide sequence of the mRNA and the nucleotide sequence of the poly(A) chain are contiguous.

**19.** The RNA according to claim 17 or 18, wherein the protein is a wild-type mitochondrial protein derived from mitochondrial DNA.

**20.** A DNA comprising a nucleotide sequence of a promoter and a nucleotide sequence complementary to the RNA defined in any one of claims 17 to 19.

**21.** A kit for detecting T10158C, which is a point mutation of the mitochondrial ND3 gene, comprising:

a wild-type detection primer DNA set consisting of a combination of a primer DNA comprising the nucleotide sequence set forth in SEQ ID NO: 2 and a primer DNA consisting of the nucleotide sequence set forth in SEQ ID NO: 4; and
a mutant detection primer DNA set consisting of a combination of a primer DNA comprising the nucleotide sequence set forth in SEQ ID NO: 2 and a primer DNA consisting of the nucleotide sequence set forth in SEQ ID NO: 10.

**22.** The kit according to claim 21, wherein the primer DNA comprising the nucleotide sequence set forth in SEQ ID NO: 2 is a primer DNA consisting of the nucleotide sequence set forth in SEQ ID NO: 3.

**23.** The kit according to claim 21, wherein the primer DNA comprising the nucleotide sequence set forth in SEQ ID NO: 2 is a primer DNA consisting of the nucleotide sequence set forth in SEQ ID NO: 3 in which one or two nucleotides at the 5' end and/or the 3' end are deleted.

**24.** The kit according to claim 21 or 23, wherein the primer DNA comprising the nucleotide sequence set forth in SEQ ID NO: 2 is a primer DNA consisting of the nucleotide sequence set forth in SEQ ID NO: 2.

# FIG. 1

tR-mRNA-tR TYPE: tR$^{G}$-mRNA(ND3)-tR$^{R}$

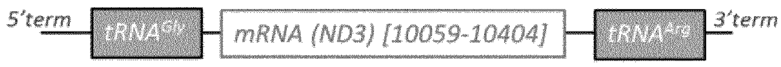


```
gaattcTAAT ACGACTCACT ATAGGGcatc tattgatgag ggtcttactc ttttagtata aatagtaccg ttaacttcca attaactagt tttgacaaca
EcoRI
ttcaaaaaag agtaATAAAC TTCGCCTTAA TTTTAATAAT CAACACCCTC CTAGCCTTAC TACTAATAAT TATTACATTT TGACTACCAC AACTCAACGG
CTACATAGAA AAATCCACCC CTTACGAGTG CGGCTTCGAC CCTATATCCC CCGCCCGCGT CCCTTTCTCC ATAAAATTCT TCTTAGTAGC TATTACCTTC
TTATTATTTG ATCTAGAAAT TGCCCTCCTT TTACCCCTAC CATGAGCCCT ACAAACAACT AACCTGCCAC TAATAGTTAT GTCATCCCTC TTATTAATCA
TCATCCTAGC CCTAAGTCTG GCCTATGAGT GACTACAAAA AGGATTAGAC TGAACCGAAT tggtatatag tttaaacaaa acgaatgatt tcgactcatt
aaattatgat aatcatattt accaaatgcc cctcatttac ataaatgata tc
EcoRV
```

7-26 : T7 PROMOTER

27-546: TARGET RNA SEQUENCE (47-114: tRNA$^{Gly}$, 115-460: ND3 mRNA, 461-525: tRNA$^{Arg}$)

# FIG. 2

ATG-mRNA-polyA TYPE : ATG-mRNA(ND3)-polyA

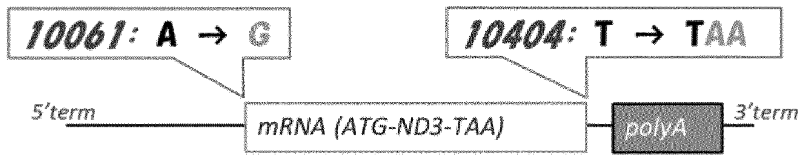


```
gaattcTAAT ACGACTCACT ATAGGGATGA ACTTCGCCTT AATTTTAATA ATCAACACCC TCCTAGCCTT ACTACTAATA ATTATTACAT TTTGACTACC
ACAACTCAAC GGCTACATAG AAAAATCCAC CCCTTACGAG TGCGGCTTCG ACCCTATATC CCCCGCCCGC GTCCCTTTCT CCATAAAATT CTTCTTAGTA
GCTATTACCT TCTTATTATT TGATCTAGAA ATTGCCCTCC TTTTACCCCT ACCATGAGCC CTACAAACAA CTAACCTGCC ACTAATAGTT ATGTCATCCC
TCTTATTAAT CATCATCCTA GCCCTAAGTC TGGCCTATGA GTGACTACAA AAAGGATTAG ACTGAACCGA ATAAAAAAAA AAAAAAAAAA AAAAAAAAAA
AAAAAAAAAA AAAAAAAAAA AAAAgatatc
```

7-26 : T7 PROMOTER

27-424 : TARGET RNA SEQUENCE (27-374: ATG-ND3-TAA mRNA, 375-424: poly A)

FIG. 3

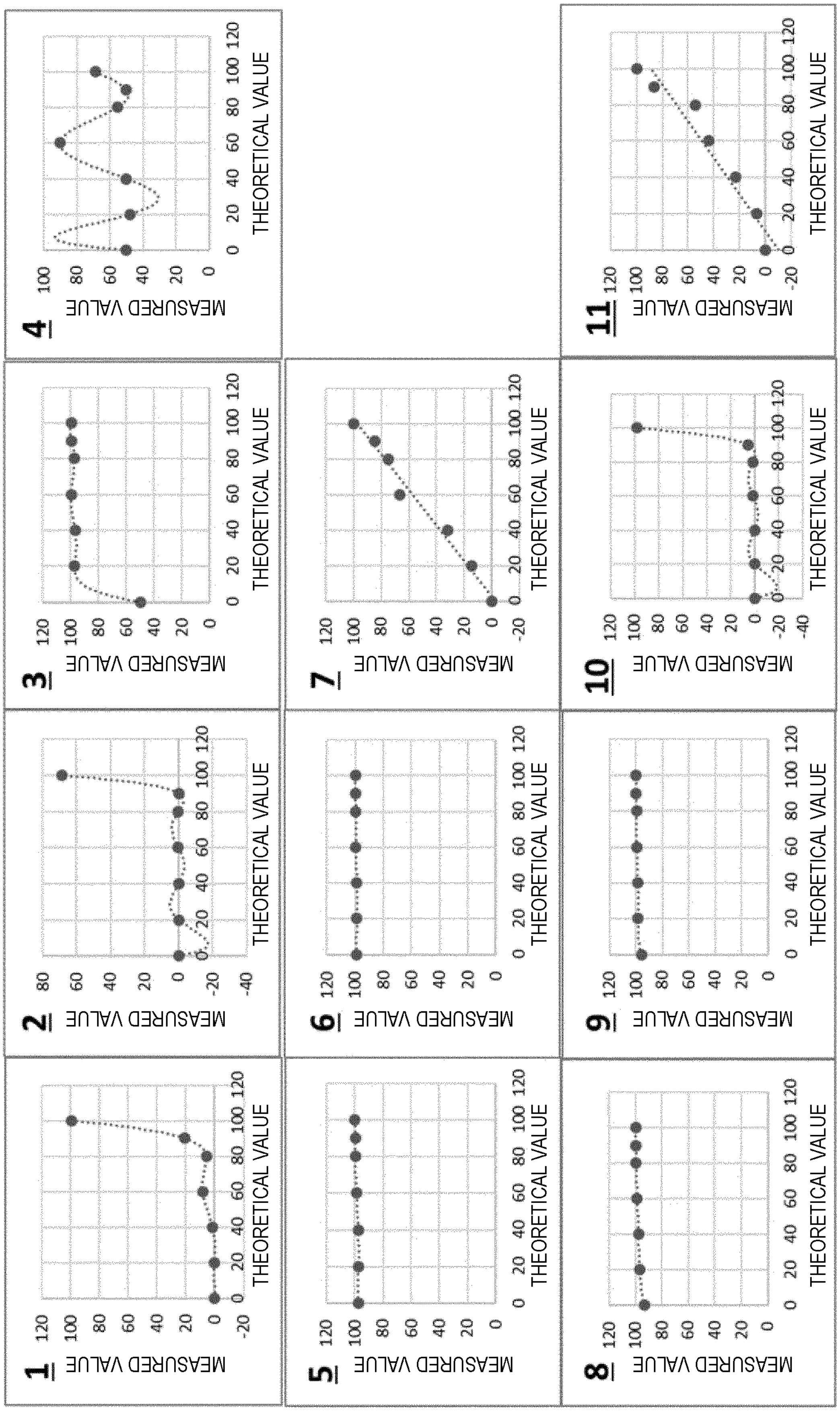

# FIG. 4

PRIMER SET 7:

$C_{Twild\ type}$: Forward (F) x Reverse (WT1)

/ $C_{Tmut\ type}$: Forward (F) x Reverse (MT1 long)

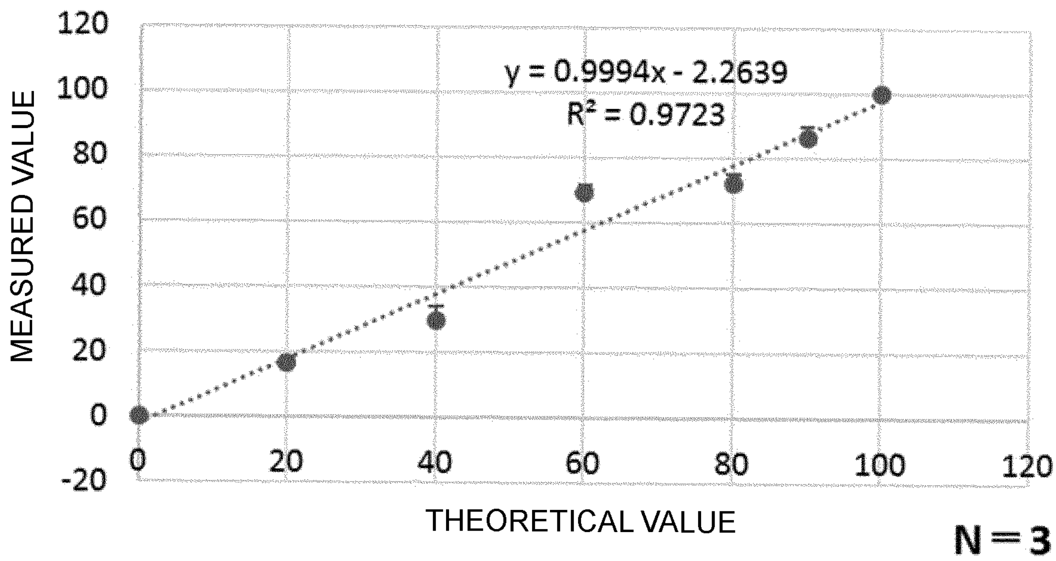


PRIMER SET 11:

$C_{Twild\ type}$: Forward (F long) x Reverse (WT1)

/ $C_{Tmut\ type}$: Forward (F long) x Reverse (MT1 long)

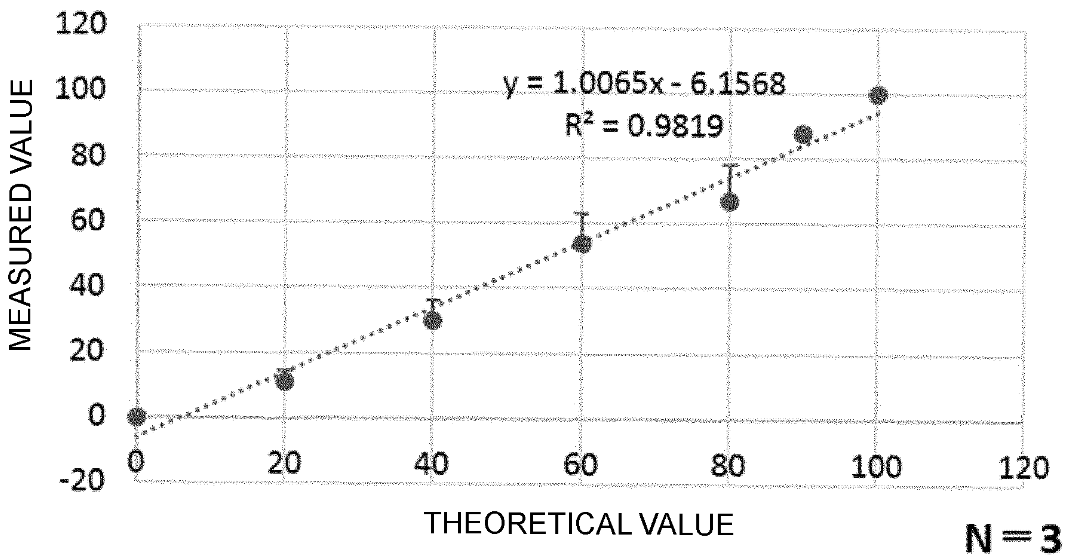

FIG. 5

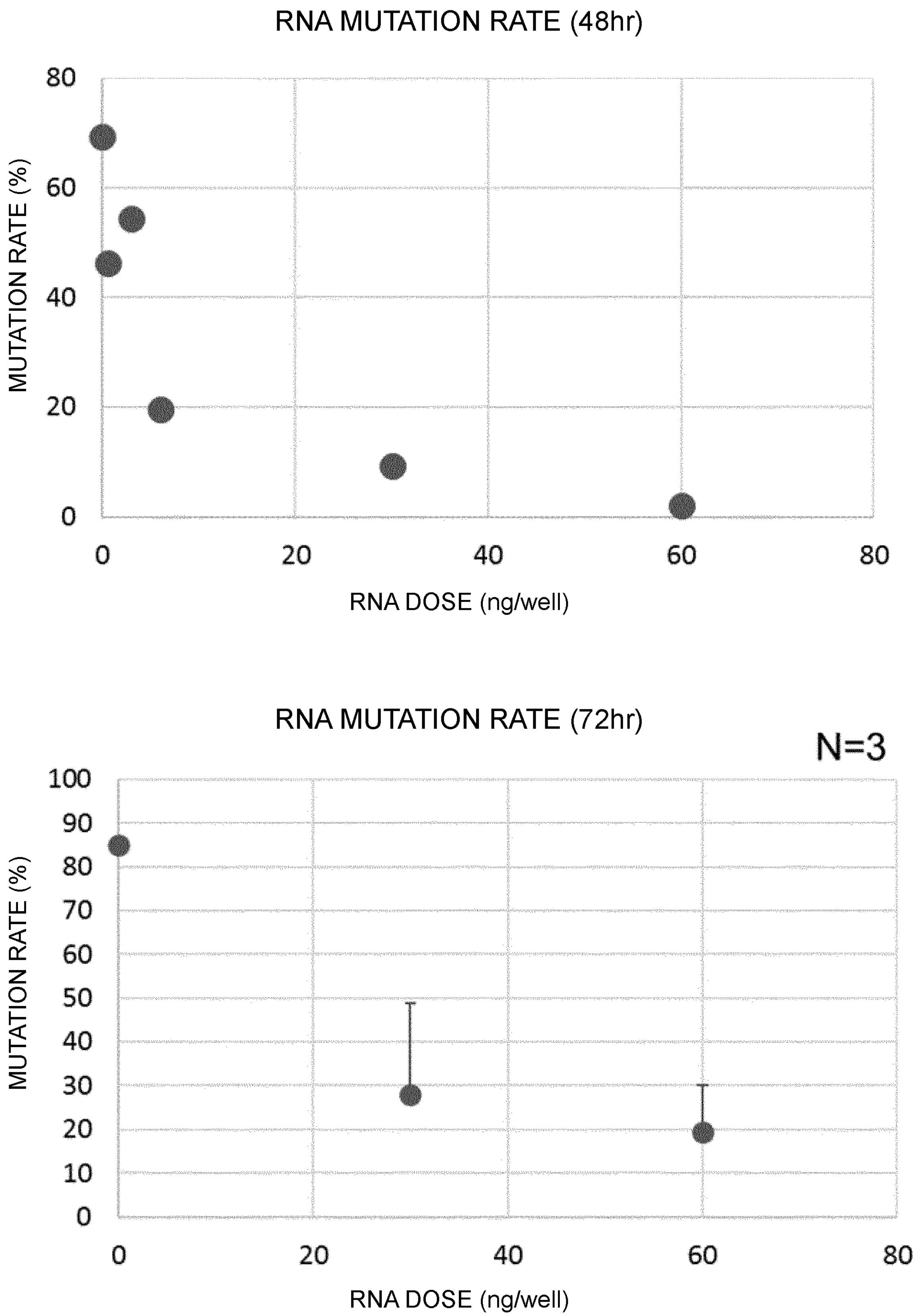
RNA MUTATION RATE (48hr)
MUTATION RATE (%)
80
60
40
20
0
0
20
40
60
80
RNA DOSE (ng/well)
RNA MUTATION RATE (72hr)
N=3
MUTATION RATE (%)
100
90
80
70
60
50
40
30
20
10
0
0
20
40
60
80
RNA DOSE (ng/well)

FIG. 6

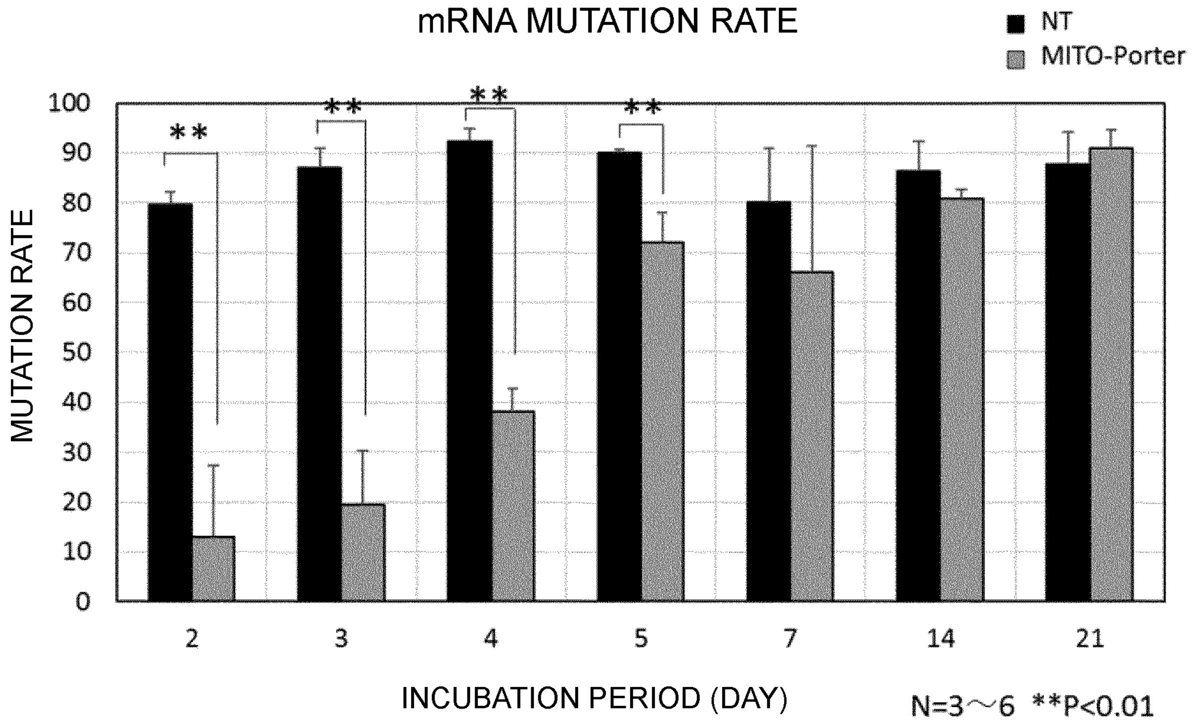
mRNA MUTATION RATE
NT
MITO-Porter
MUTATION RATE
100
90
80
70
60
50
40
30
20
10
0
**
**
**
**
2
3
4
5
7
14
21
INCUBATION PERIOD (DAY)
N=3～6 **P<0.01

FIG. 7

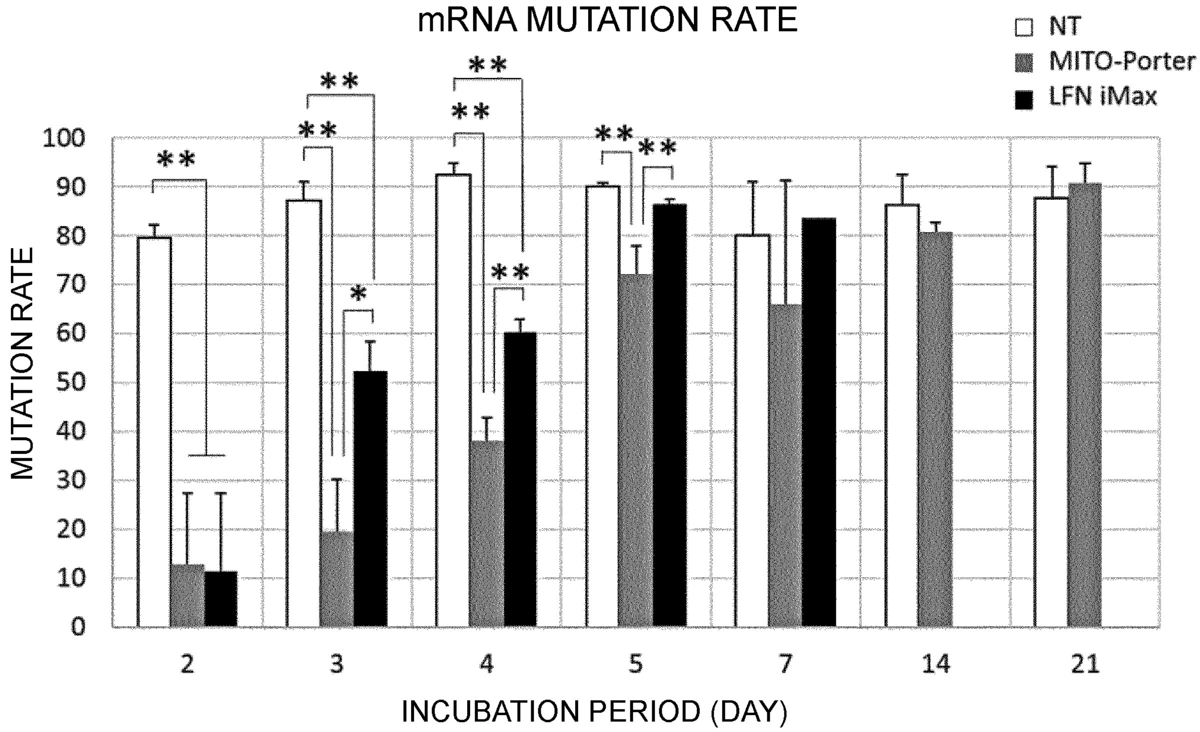
mRNA MUTATION RATE
NT
MITO-Porter
LFN iMax
MUTATION RATE
100
90
80
70
60
50
40
30
20
10
0
**
**
**
*
**
**
**
**
**
2
3
4
5
7
14
21
INCUBATION PERIOD (DAY)

FIG. 8

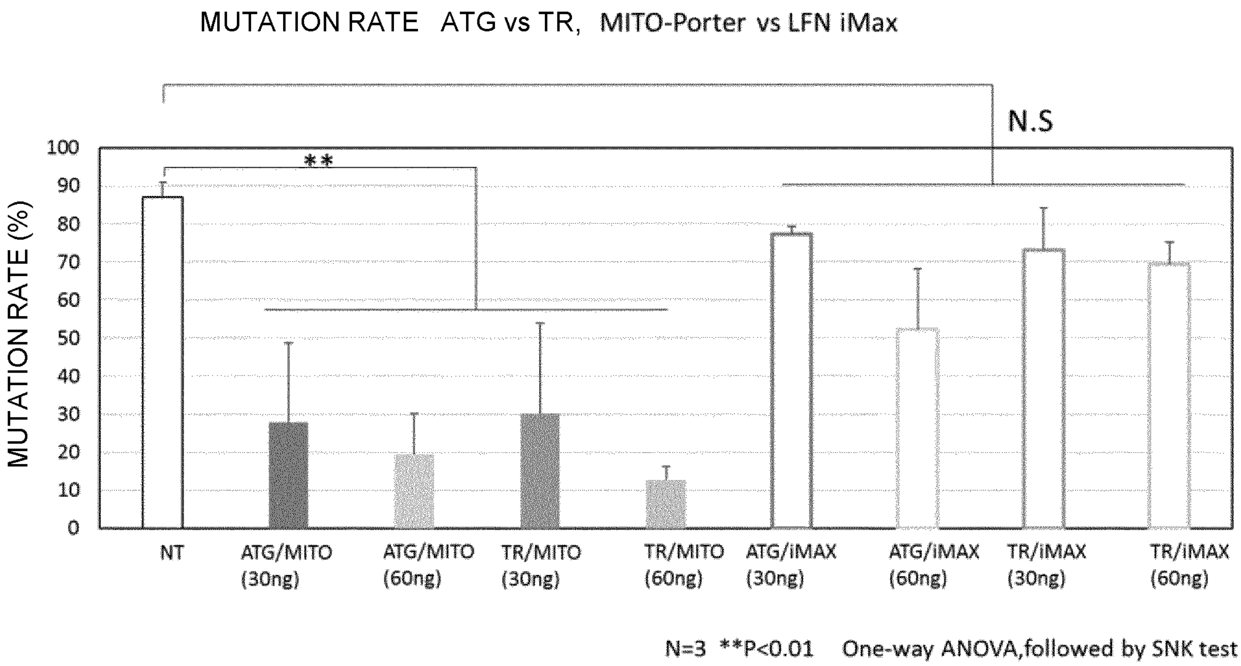
MUTATION RATE ATG vs TR, MITO-Porter vs LFN iMax
N.S
**
MUTATION RATE (%)
100
90
80
70
60
50
40
30
20
10
0
NT
ATG/MITO (30ng)
ATG/MITO (60ng)
TR/MITO (30ng)
TR/MITO (60ng)
ATG/iMAX (30ng)
ATG/iMAX (60ng)
TR/iMAX (30ng)
TR/iMAX (60ng)
N=3 **P<0.01 One-way ANOVA,followed by SNK test

FIG. 9

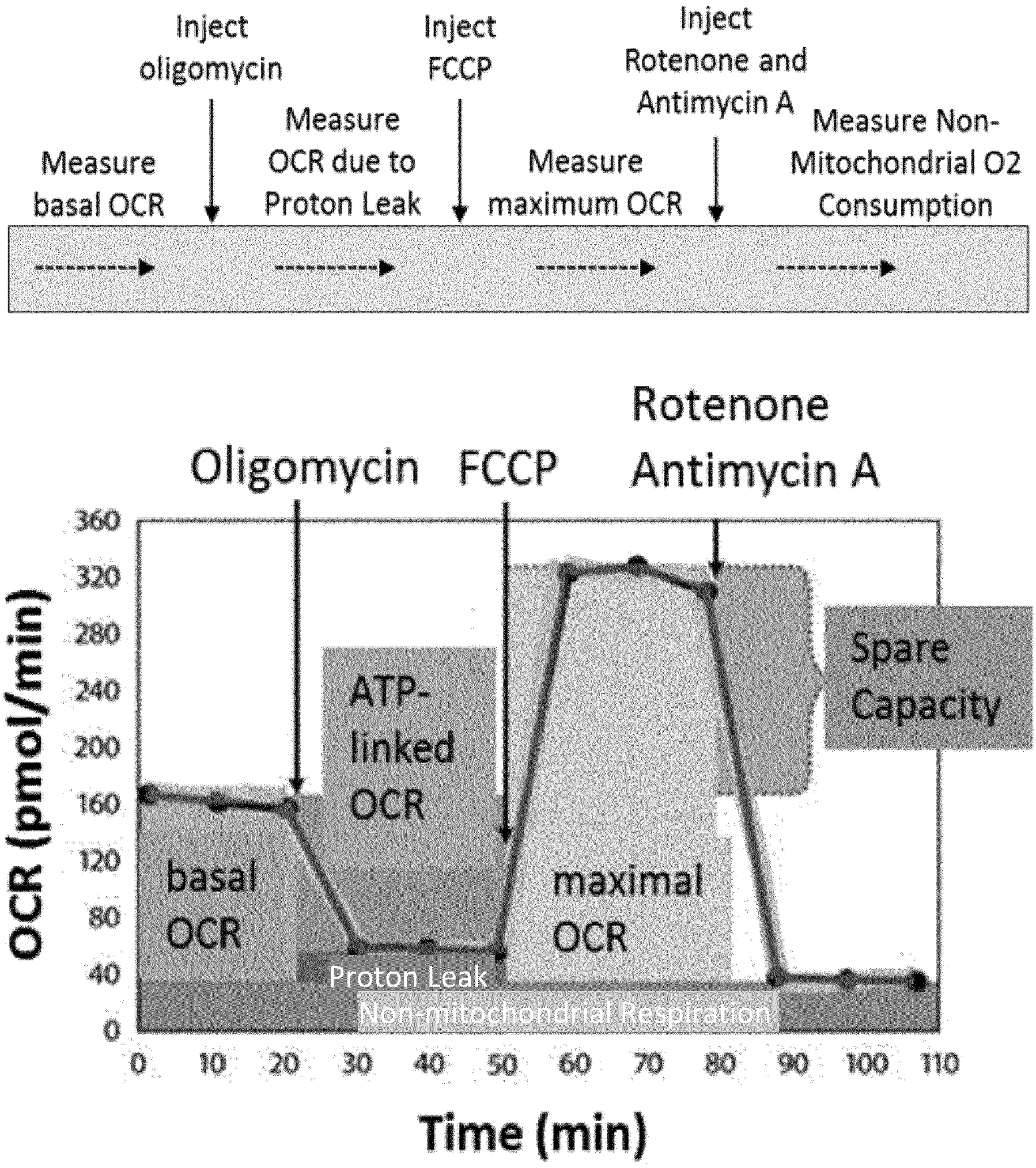
Inject
oligomycin
Inject
FCCP
Inject
Rotenone and
Antimycin A
Measure
basal OCR
Measure
OCR due to
Proton Leak
Measure
maximum OCR
Measure Non-
Mitochondrial O2
Consumption
Oligomycin
FCCP
Rotenone
Antimycin A
OCR (pmol/min)
360
320
280
240
200
160
120
80
40
0
ATP-
linked
OCR
Spare
Capacity
basal
OCR
maximal
OCR
Proton Leak
Non-mitochondrial Respiration
0
10
20
30
40
50
60
70
80
90
100
110
Time (min)

FIG. 10

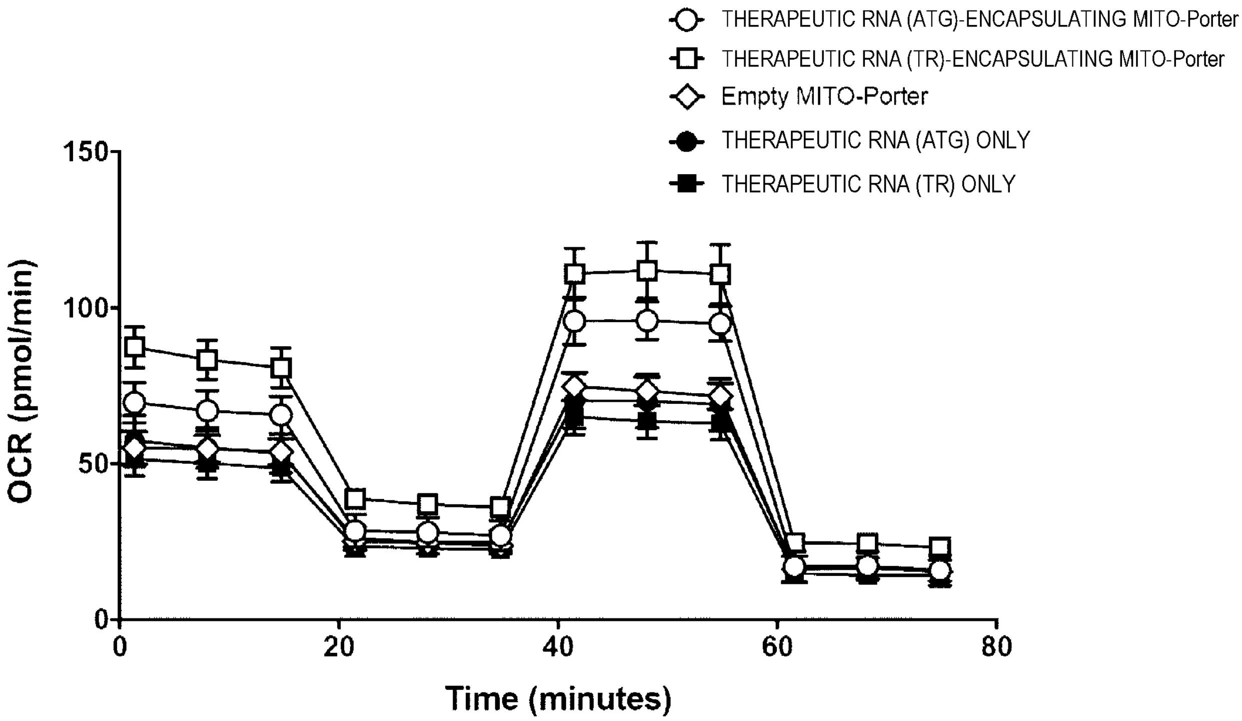
THERAPEUTIC RNA (ATG)-ENCAPSULATING MITO-Porter
THERAPEUTIC RNA (TR)-ENCAPSULATING MITO-Porter
Empty MITO-Porter
THERAPEUTIC RNA (ATG) ONLY
THERAPEUTIC RNA (TR) ONLY
150
100
50
0
OCR (pmol/min)
0
20
40
60
80
Time (minutes)

FIG. 11

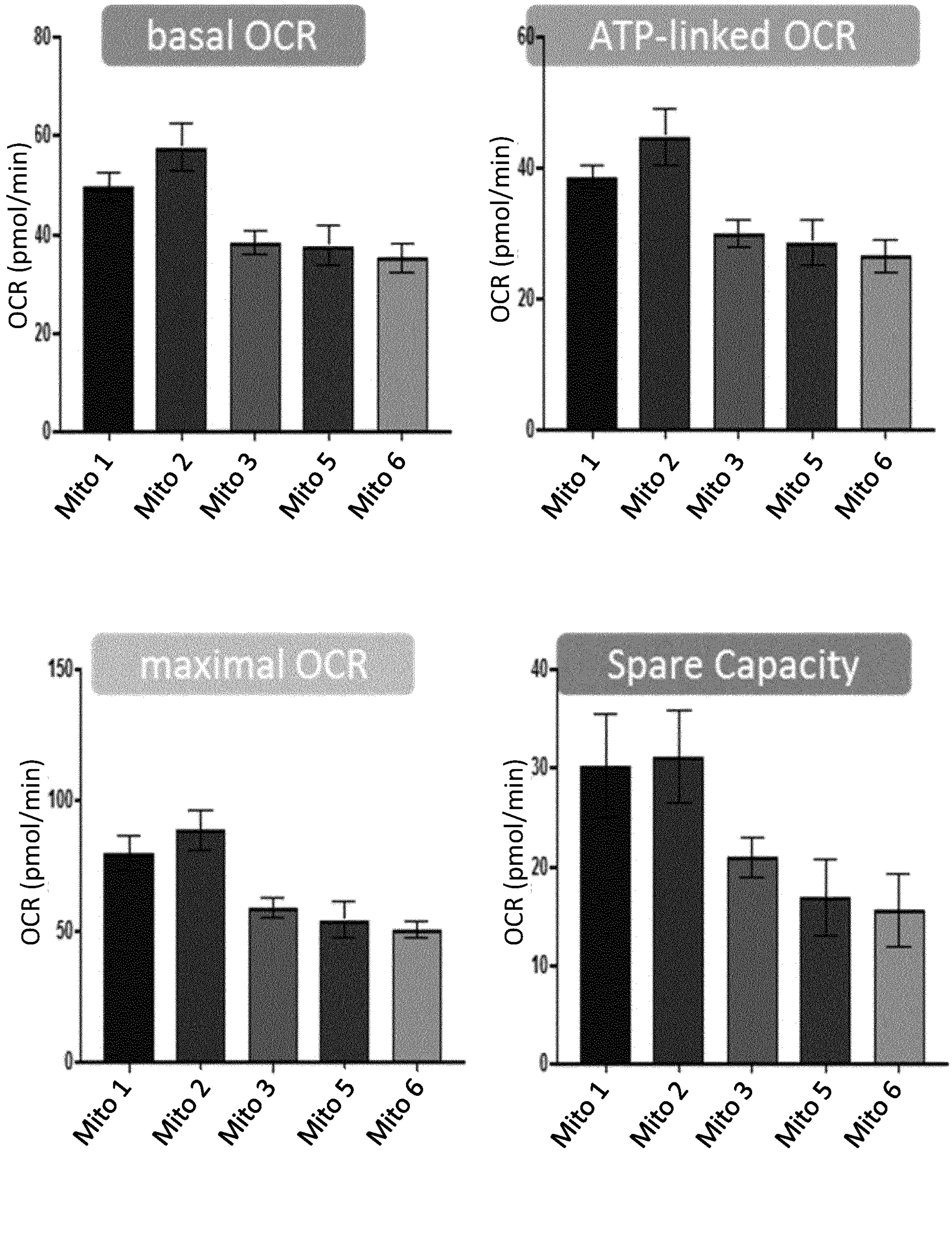
basal OCR
OCR (pmol/min)
80
60
40
20
0
Mito 1
Mito 2
Mito 3
Mito 5
Mito 6
ATP-linked OCR
OCR (pmol/min)
60
40
20
0
Mito 1
Mito 2
Mito 3
Mito 5
Mito 6
maximal OCR
OCR (pmol/min)
150
100
50
0
Mito 1
Mito 2
Mito 3
Mito 5
Mito 6
Spare Capacity
OCR (pmol/min)
40
30
20
10
0
Mito 1
Mito 2
Mito 3
Mito 5
Mito 6

FIG. 12

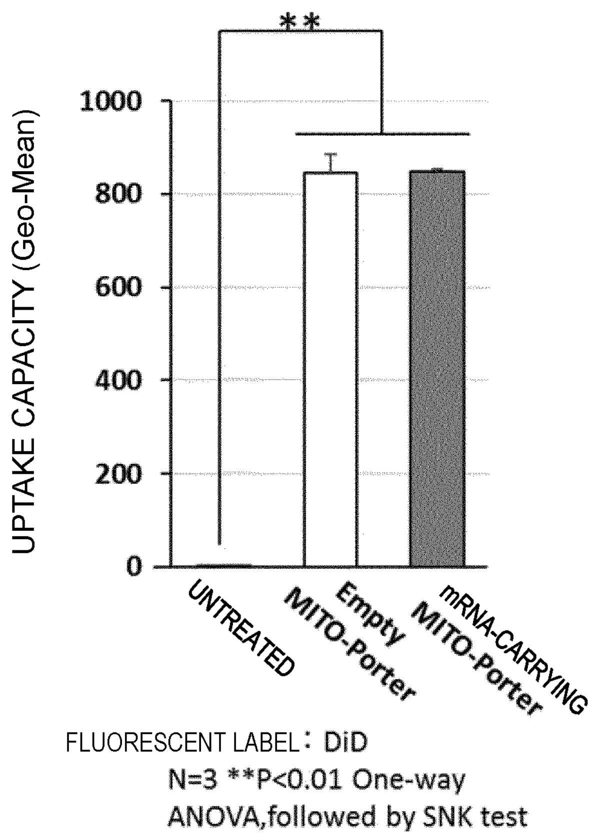

**
1000
800
600
400
200
0
UPTAKE CAPACITY (Geo-Mean)
UNTREATED
Empty MITO-Porter
mRNA-CARRYING MITO-Porter
FLUORESCENT LABEL：DiD
N=3 **P<0.01 One-way
ANOVA,followed by SNK test


FIG. 13

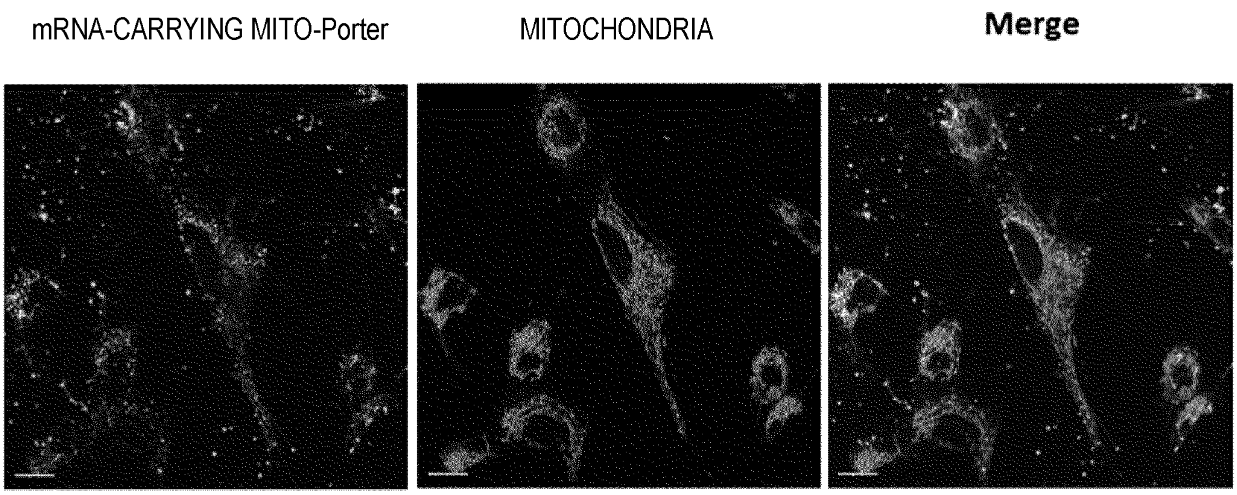

mRNA-CARRYING MITO-Porter
MITOCHONDRIA
Merge

**INTERNATIONAL SEARCH REPORT**

International application No.
PCT/JP2019/007145

A. CLASSIFICATION OF SUBJECT MATTER
Int.Cl. A61K48/00(2006.01)i, A61K9/127(2006.01)i, A61P43/00(2006.01)i, C12N15/11(2006.01)i, C12N15/12(2006.01)i, C12N15/88(2006.01)i, C12Q1/6883(2018.01)i, G01N33/53(2006.01)i

B. FIELDS SEARCHED

Minimum documentation searched (classification system followed by classification symbols)
Int.Cl. A61K48/00, A61K9/127, A61P43/00, C12N15/11, C12N15/12, C12N15/88, C12Q1/6883, G01N33/53

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/REGISTRY/MEDLINE/EMBASE/BIOSIS (STN), JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| X<br><br>Y<br>A | LYRAWATI, D. et al., "Expression of GFP in the Mitochondrial Compartment Using DQAsome-Mediated Delivery of an Artificial Mini-mitochondrial Genome", Pharm Res, 2011, 28, pp. 2848-2862, abstract, fig. 1, page 2849, right column, line 20 to page 2851, left column, line 12, page 2852, left column, lines 8-43 | 1-2, 7-8, 11-13, 16<br>1-20<br>21-24 |
| Y<br>A | WO 2017/090763 A1 (NATIONAL UNIVERSITY CORPORATION HOKKAIDO UNIVERSITY) 01 June 2017, claims 1, 11, paragraphs [0022], [0023], [0054] & US 2018/0362999 A1, claim 1, paragraphs [0041], [0042], [0073] & EP 3382020 A1 | 1-20<br>21-24 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 18 April 2019 (18.04.2019) | 07 May 2019 (07.05.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/007145

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | ADHYA, S. et al., "Mitochondrial gene therapy: The tortuous path from bench to bedside", Mitochondrion, 2011, 11, pp. 839-844, fig. 1, page 842, right column to page 843, left column, column "3. 5. Mitochondriotropic RNA carriers" | 1-20<br>21-24 |
| Y<br>A | KOUGA, T. et al., "Japanese Leigh syndrome case treated with EPI-743", Brain & Development, September 2017, 40, pp. 145-149, abstract, fig. 3 | 21-24<br>1-20 |
| Y<br>A | 中村道三他, mutation-specific PCRによるmitDNA点変異検出法, Nippon Rinsho, 1997, vol. 55, no. 12, 3277-3281, page 3278, right column "a. Design of primer", non-official translation (NAKAMURA, Michikazu et al., "Method for detecting mitDNA point mutation by means of mutation-specific PCR") | 21-24<br>1-20 |
| A | YAMADA, Y. et al., "A Dual-Ligand Liposomal System Composed of a Cell-Penetrating Peptide and a Mitochondrial RNA Aptamer Synergistically Facilitates Cellular Uptake and Mitochondrial Targeting", Journal of Pahrmaceutical Sciences, 2016, 105, pp. 1705-1713 | 1-24 |
| A | JP 2007-524392 A (GENCIA CORPORATION) 30 August 2007 & US 2007/0196334 A1 & WO 2005/001062 A2 & EP 1644496 A2 | 1-24 |
| P, A | JANG, YH et al., "Recent Advances in Mitochondria-Targeted Gene Delivery", Molecules, September 2018, 23, 2316 | 1-24 |

Form PCT/ISA/210 (continuation of second sheet) (January 2015)

**INTERNATIONAL SEARCH REPORT**

International application No.
PCT/JP2019/007145

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.
2. ☒ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest and, where applicable, the payment of a protest fee.
☐ The additional search fees were accompanied by the applicant's protest but the applicable protest fee was not paid within the time limit specified in the invitation.
☐ No protest accompanied the payment of additional search fees.

Form PCT/ISA/210 (continuation of first sheet (2)) (January 2015)

INTERNATIONAL SEARCH REPORT

International application No.
PCT/JP2019/007145

<Continuation of Box No. III>

(Invention 1) Claims 1-16

Claims 1-16 have the special technical feature of "a mitochondrial-directed lipid membrane structure formed by enclosing a nucleic acid represented by any of the following a) to d):

a) RNA which includes a base sequence of a first mitochondrial tRNA, a base sequence of mRNA encoding a target protein, and a base sequence of a second mitochondrial tRNA in this order, wherein the base sequence of mRNA has one or a plurality of UGAs as a tryptophan codon;

b) DNA which includes a base sequence of a promoter and a complementary base sequence in RNA of a);

c) RNA which includes a base sequence of mRNA encoding a target protein and a base sequence of a poly(A) chain present at the 3' end thereof, wherein the base sequence of mRNA has one or a plurality of UGAs as a tryptophan codon, AUG as a start codon, and UAA as a stop codon; and

d) DNA which includes a base sequence of a promoter and a complementary base sequence in RNA of c)", and thus are classified as invention 1.

(Invention 2) Claims 17-20

Claims 17-20 cannot be considered to have technical features identical or corresponding to those of claim 1 classified as invention 1.

Also, claims 17-20 are not dependent on claim 1. Furthermore, claims 17-20 are not substantially identical or equivalent to any of the claims classified as invention 1.

Thus, claims 17-20 cannot be classified as invention 1.

In addition, claims 17-20 have the special technical feature of "RNA which includes a base sequence of mRNA encoding a target protein and a base sequence of a poly(A) chain in the 3' end thereof, wherein the base sequence of mRNA has one or a plurality of UGAs as a tryptophan codon, AUG as a start codon, and UAA as a stop codon", and thus are classified as invention 2.

(Invention 3) Claims 21-24

Claims 21-24 cannot be considered to have a technical feature identical or corresponding to that of claim 1 classified as invention 1 or claim 17 classified as invention 2.

Furthermore, claims 21-24 are not dependent on claim 1 and claim 17. In addition, claims 21-24 are not substantially identical or equivalent to any of the claims classified as invention 1 or invention 2.

Thus, claims 21-24 cannot be classified as invention 1 or invention 2.

In addition, claims 21-24 have the special technical feature of being "a kit for detecting T10158C, which is a point mutation of a mitochondrial ND3 gene, the kit including: a primer DNA set for detecting wild-types which is made up of a combination of a primer DNA including the base sequence represented by SEQ ID: 2 and a primer DNA which is made up of the base sequence represented by SEQ ID: 4; and a primer DNA set for detecting mutant-types which is made up of a combination of a primer DNA including the base sequence represented by SEQ ID: 2 and a primer DNA which is made up of the base sequence represented by SEQ ID: 10", and thus are classified as invention 3.

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20150225740 A
- WO 2017090763 A
- US 20180362999 A
- JP 5067733 B
- WO 2006095837 A


### Non-patent literature cited in the description


- **YU,H. et al.** *Proc. Natl. Acad. Sci. USA,* 2012, vol. 109, E1238-47
- **SHAHEEN et al.** *Biomaterials,* 2011, vol. 32, 6342-6350
- Japanese Pharmacopoeia
- **V. VENEGAS et al.** *Methods Mol. Biol.,* 2012, vol. 837, 313-326
- **MCFARLAND R. et al.** *Ann Neurol.,* 2004, vol. 55 (1), 58-64
- **ABE et al.** *J. Pharm. Sci.,* 2016, vol. 105, 734-740

SEQUENCE LISTING

<110> NATIONAL UNIVERSITY CORPORATION HOKKAIDO UNIVERSITY
JICHI MEDICAL UNIVERSITY

<120> NUCLEIC ACID FOR EXPRESSION OF PROTEIN IN MITOCHONDRIA, LIPID MEMBRANE STRUCTURE HAVING SAID NUCLEIC ACID ENCAPSULATED THEREIN AND THEIR USE

<130> P17HU09WO

<160> 14

<170> PatentIn version 3.5

<210> 1
<211> 27
<212> PRT
<213> Artificial Sequence

<220>
<223> synthesized peptide

<400> 1

```
Trp Glu Ala Lys Leu Ala Lys Ala Leu Ala Lys Ala Leu Ala Lys His
1               5                   10                  15


Leu Ala Lys Ala Leu Ala Lys Ala Leu Lys Ala
            20                  25
```

<210> 2
<211> 20
<212> DNA
<213> Artificial Sequence

<220>
<223> synthesized oligonucleotide

<400> 2
caacaccctc ctagccttac 20

<210> 3
<211> 24
<212> DNA
<213> Artificial Sequence

<220>
<223> synthesized oligonucleotide

<400> 3
atcaacaccc tcctagcctt acta 24

<210> 4
<211> 20
<212> DNA
<213> Artificial Sequence

<220>

<223> synthesized oligonucleotide

<400> 4
ccgcactcgt aaggggtgca 20

<210> 5
<211> 20
<212> DNA
<213> Artificial Sequence

<220>
<223> synthesized oligonucleotide

<400> 5
ccgcactcgt aaggggtcca 20

<210> 6
<211> 20
<212> DNA
<213> Artificial Sequence

<220>
<223> synthesized oligonucleotide

<400> 6
ccgcactcgt aaggggtgcg 20

<210> 7
<211> 20
<212> DNA
<213> Artificial Sequence

<220>
<223> synthesized oligonucleotide

<400> 7
ccgcactcgt aaggggtccg 20

<210> 8
<211> 20
<212> DNA
<213> Artificial Sequence

<220>
<223> synthesized oligonucleotide

<400> 8
ccgcactcgt aaggggtggg 20

<210> 9
<211> 22
<212> DNA
<213> Artificial Sequence

<220>
<223> synthesized oligonucleotide

<400> 9

agccgcactc gtaaggggtg gg 22

<210> 10
<211> 22
<212> DNA
<213> Artificial Sequence

<220>
<223> synthesized oligonucleotide

<400> 10
agccgcactc gtaaggggtg cg 22

<210> 11
<211> 552
<212> DNA
<213> Artificial Sequence

<220>
<223> synthesized oligonucleotide

<400> 11
gaattctaat acgactcact atagggcatc tattgatgag ggtcttactc ttttagtata 60

aatagtaccg ttaacttcca attaactagt tttgacaaca ttcaaaaaag agtaataaac 120

ttcgccttaa ttttaataat caacaccctc ctagccttac tactaataat tattacattt 180

tgactaccac aactcaacgg ctacatagaa aaatccaccc cttacgagtg cggcttcgac 240

cctatatccc ccgcccgcgt ccctttctcc ataaaattct tcttagtagc tattaccttc 300

ttattatttg atctagaaat tgccctcctt ttacccctac catgagccct acaaacaact 360

aacctgccac taatagttat gtcatccctc ttattaatca tcatcctagc cctaagtctg 420

gcctatgagt gactacaaaa aggattagac tgaaccgaat tggtatatag tttaaacaaa 480

acgaatgatt tcgactcatt aaattatgat aatcatattt accaaatgcc cctcatttac 540

ataaatgata tc 552

<210> 12
<211> 430
<212> DNA
<213> Artificial Sequence

<220>
<223> synthesized oligonucleotide

<400> 12
gaattctaat acgactcact atagggatga acttcgcctt aattttaata atcaacaccc 60

tcctagcctt actactaata attattacat tttgactacc acaactcaac ggctacatag 120

aaaaatccac cccttacgag tgcggcttcg accctatatc ccccgcccgc gtccctttct 180

ccataaaatt cttcttagta gctattacct tcttattatt tgatctagaa attgccctcc 240

ttttacccct accatgagcc ctacaaacaa ctaacctgcc actaatagtt atgtcatccc 300

tcttattaat catcatccta gccctaagtc tggcctatga gtgactacaa aaaggattag 360

actgaaccga ataaaaaaaa aaaaaaaaaa aaaaaaaaaa aaaaaaaaaa aaaaaaaaaa 420

aaaagatatc 430

<210> 13
<211> 520
<212> RNA
<213> Artificial Sequence

<220>
<223> synthesized oligonucleotide

<400> 13
caucuauuga ugagggucuu acucuuuuag uauaaauagu accguuaacu uccaauuaac 60

uaguuuugac aacauucaaa aaagaguaau aaacuucgcc uuaauuuuaa uaaucaacac 120

ccuccuagcc uuacuacuaa uaauuauuac auuuugacua ccacaacuca acggcuacau 180

agaaaaaucc accccuuacg agugcggcuu cgacccuaua ucccccgccc gcgucccuuu 240

cuccauaaaa uucuucuuag uagcuauuac cuucuuauua uuugaucuag aaauugcccu 300

ccuuuuaccc cuaccaugag cccuacaaac aacuaaccug ccacuaauag uuaugucauc 360

ccucuuauua aucaucaucc uagcccuaag ucuggccuau gagugacuac aaaaaggauu 420

agacugaacc gaauugguau auaguuuaaa caaaacgaau gauuucgacu cauuaaauua 480

ugauaaucau auuuaccaaa ugccccucau uuacauaaau 520

<210> 14
<211> 398
<212> RNA
<213> Artificial Sequence

<220>
<223> synthesized oligonucleotide

<400> 14
augaacuucg ccuuaauuuu aauaaucaac acccuccuag ccuuacuacu aauaauuauu 60

acauuuugac uaccacaacu caacggcuac auagaaaaau ccaccccuua cgagugcggc 120

uucgacccua uaucccccgc ccgcgucccu uucuccauaa aauucuucuu aguagcuauu 180

accuucuuau uauuugaucu agaaauugcc cuccuuuuac cccuaccaug agcccuacaa 240

acaacuaacc ugccacuaau aguuauguca ucccucuuau uaaucaucau ccuagcccua 300

agucuggccu augagugacu acaaaaagga uuagacugaa ccgaauaaaa aaaaaaaaaa 360

aaaaaaaaaa aaaaaaaaaa aaaaaaaaaa aaaaaaaa 398